# FASCICULE DE BREVET EUROPEEN

(11) **EP 2 421 495 B1**
(45) Date de publication et mention de la délivrance du brevet: **10.02.2016**
(21) Numéro de dépôt: 10718245.3
(22) Date de dépôt: 23.04.2010
(51) Int. Cl.: A61K 8/91, A61K 9/107, A61K 8/06, A61K 8/11, A61K 8/49, A61K 8/73, A61K 8/92, A61Q 17/04, A61K 47/40

(54) **PROCÉDÉ DE FORMATION D'ÉMULSIONS À BASE DE POLYMÈRES DE CYCLODEXTRINE ET DE COMPOSÉS LIPOPHILES, ÉMULSIONS AINSI OBTENUES, ET COMPOSITIONS COMPRENANT LESDITES ÉMULSIONS**
VERFAHREN ZUR BILDUNG VON EMULSIONEN AUS CYCLODEXTRIN-POLYMER UND LIPOPHILEN VERBINDUNGEN, RESULTIERENDE EMULSIONEN UND DIESE EMULSIONEN ENTHALTENDE ZUSAMMENSETZUNGEN
METHOD FOR FORMING CYCLODEXTRIN POLYMER AND LIPOPHILIC COMPOUND EMULSIONS, RESULTING EMULSIONS, AND COMPOSITIONS INCLUDING SAID EMULSIONS

(30) Priorité: 23.04.2009 FR 0901983; 07.09.2009 FR 0956088
(43) Date de publication de la demande: 29.02.2012
(73) Titulaire: Centre National de la Recherche Scientifique (CNRS), 75794 Paris Cedex 16 (FR); Université Paris-Sud, 91400 Orsay (FR)
(72) Inventeur: LAZA-KNOERR, Anca-Lucia, F-91940 Les Ulis (FR); GREF, Ruxandra, F-91270 Verrieres-le-Buisson (FR); AMIEL, Catherine, F-94240 L'Hay-les-Roses (FR); COUVREUR, Patrick, F-91140 Villebon-sur-Yvette (FR)
(74) Mandataire: Novagraaf Technologies
(86) Numéro de dépôt international: PCT/FR2010/000330
(87) Numéro de publication internationale: WO 2010/122246

(56) Documents cités:
- EP-A2- 0 366 154
- EP-A2- 0 875 240
- WO-A1-2008/003685
- US-A1- 2002 019 369
- DATABASE WPI Week 198647 9 octobre 1986 (1986-10-09) Thomson Scientific, London, GB; AN 1986-308506 XP002573507, "Skin cosmetic compsn. - with water-insoluble, oil-soluble components blended in cosmetics as clathrate cpds. with cyclodextrin polymer", -& JP 61 227517 A (LION CORP.) 9 octobre 1986 (1986-10-09) cité dans la demande
- DUCHÊNE D. ET AL.: "Cyclodextrins and emulsions.", INTERNATIONAL JOURNAL OF PHARMACEUTICS, vol. 266, 2003, pages 85-90, XP002573508, cité dans la demande
- DATABASE BIOSIS BIOSCIENCES INFORMATION SERVICE, PHILADELPHIA, PA, US; septembre 2008 (2008-09), Inoue M. et al.: "Emulsion preparation using beta-cyclodextrin and its derivatives acting as an emulsifier", XP002573509, Database accession no. PREV200800590531 cité dans la demande & CHEMICAL & PHARMACEUTICAL BULLETIN (TOKYO), vol. 56, no. 9, septembre 2008 (2008-09), pages 1335-1337, ISSN: 0009-2363
- T.OOYA ET AL.: "Hydrogels having tubular alpha-cyclodextrin structure: effect of nano-tube structure on long alkyl chain partitions", SCIENCE AND TECHNOLOGY OF ADVANCED MATERIALS, vol. 4, no. 1, 2003, pages 39-42, XP009143798,

## Description

### Domaine Technique

La présente invention concerne un procédé de préparation d'émulsions à base de polymères de cyclodextrine et/ou de polymères hydrophiles portant des cyclodextrines d'une part, et de composés lipophiles d'autre part, les émulsions présentant une stabilité remarquable. L'invention concerne également les émulsions ainsi obtenues, c'est-à-dire des émulsions stabilisées par un complexe d'inclusion non-covalent et non-cristallin formé (i) d'un polymère à base d'unités cyclodextrine ou d'un polymère hydrophile portant des cyclodextrines et (ii) d'un composé lipophile. L'invention a également trait à l'utilisation de ces émulsions dans le domaine cosmétique, agro-alimentaire ou pharmaceutique.

Dans la description ci-dessous, les références entre crochets ([ ]) renvoient à la liste des références présentée après les exemples.

### Etat de la Technique

Les émulsions sont très utilisées dans des nombreux domaines techniques, du simple fait qu'elles permettent la dispersion durable d'au moins deux phases non-miscibles liquide-liquide généralement par l'intermédiaire d'un agent de surface (tensioactif).

Il existe deux grandes catégories d'émulsions : les émulsions huile-dans-l'eau (émulsions obtenues par voie directe) et les émulsions eau-dans-l'huile (émulsions obtenues par voie inverse). Dans le premier cas, la phase continue est l'eau (dans cette phase sont dispersées des gouttelettes de l'huile) ; et dans le second cas la phase continue est l'huile dans laquelle sont dispersées des gouttelettes d'eau. Le type d'une émulsion simple, directe ou inverse, est fixé au premier ordre par la solubilité préférentielle du tensioactif dans l'une ou l'autre phase. Le recours à un tensioactif hydrosoluble permet de former préférentiellement une émulsion directe, alors qu'un tensioactif liposoluble favorise une émulsion inverse.

De nombreuses compositions cosmétiques sont préparées à base d'émulsions (typiquement des émulsions simples) stabilisées par des tensioactifs.

Par exemple, EP 0 685 227 **[1]** propose un système très complexe de compositions cosmétiques antisolaires, du type comprenant, une phase continue aqueuse, un système protecteur capable de filtrer les rayons UV (Parsol® MCX, TiO₂, dérivées de benzophenanone, etc.), un tensioactif, des solvants organiques (alcools et polyols inférieurs) et au moins un polymère ou plus particulièrement un copolymère réticulé (acrylates d'alkyle, acétate de vinyle).

FR 2 858 777 **[2],** propose pour sa part, une émulsion huile- dans -l'eau stable contenant au moins un corps gras (esters d'acides gras, les cires, les beurres, les huiles naturelles -végétale, animale, d'origine marine-, synthétiques ou minérales, les huiles hydrogénées et leurs mélanges), au moins un tensioactif (les esters d'acides gras de poly glycérol comme les éthoxylés, les éthoxylats d'alcools), au moins un co-tensioactif (polyols), et de l'eau.

Cependant, l'utilisation de tensioactifs dans des produits destinés à être administrés à l'homme (que ce soit sous forme topique, orale, ou autre) peut être problématique. En effet, les tensioactifs peuvent endommager les membranes cellulaires. Ainsi, des efforts sont consentis, notamment dans le domaine cosmétique, pour réduire les effets potentiellement nocifs des tensioactifs, voire éliminer le recours aux tensioactifs.

Dans cette optique, le recours aux cyclodextrines a été envisagé.

Par exemple, Duchêne et coll. (« Cyclodextrins and emulsions », International Journal of Pharmaceutics, 266 (2003), 85-90 **[3])** et Yu et coll. (« Effect of camphor/cyclodextrin complexation on the stability of O/W/O multiple emulsions », International Journal of Pharmaceutics, 261 (2003), 1-8 **[4])** ont proposé la formulation d'émulsions multiples en utilisant des monomères α-, β- et γ- cyclodextrines comme agent stabilisant. Deux des cyclodextrines (α-, β-) présentent des propriétés stabilisantes lors de la préparation d'une émulsion multiple (double) ; la cavité plus large de la γ- cyclodextrine étant défavorable à la stabilisation du fait de sa taille trop importante pour conduire à une interaction optimale avec les chaines alkyle).

D'autres émulsions à base des monomères de cyclodextrines ont également été proposées par Inoue et coll. (« Emulsion preparation using β- cyclodextrin and its derivates acting as an emulsifier », Chem. Pharm. Bull, 56 (9), (2008), 1335-1337 **[5],** "Formulation and characterisation of emulsions using β- cyclodextrin as an emulsifier", Chem. Pharm. Bull, 56 (5), (2008), 668- 671, **[6]** et "Preparation and characterisation of n-alkane / water emulsion stabilized by cyclodextrine", Journal of oleo science, 58, (2), (2009), 85-90, **[7]).** Les résultats indiquent que l'ajout d'une cyclodextrine (monomère-α, -β et -γ) d'une part entraine la formation d'un précipité et d'autre part, l'adsorption de celui-ci à l'interface huile-eau est nécessaire dans certains cas pour stabiliser les émulsions.

Le document WO 2008/ 003685 **[8]** propose une émulsion à base de monomères de cyclodextrines éventuellement modifiées, un polysaccharide modifié, une substance grasse et de l'eau.

Dans tous les cas, une émulsion peut être préparée, mais les monomères de cyclodextrine ne permettent pas de la stabiliser durablement (un précipité se forme).

Il y a donc un besoin de développer des procédés capables de stabiliser des émulsions sans avoir recours à des tensioactifs.

### Exposé de l'Invention

De façon tout à fait inattendue et surprenante, les inventeurs ont découvert qu'il est possible de réaliser des émulsions remarquablement stables, sans avoir pour cela à mettre en oeuvre des solvants organiques, d'agents tensioactifs, de co-tensioactifs ou d'autres additifs organiques synthétiques.

Plus précisément, les inventeurs ont découvert que l'utilisation d'un complexe d'inclusion non-covalent et non-cristallin formé (i) d'un polymère **I** à base d'unités cyclodextrine ou d'un polymère hydrophile **II** portant des cyclodextrines et (ii) d'un composé lipophile permet d'obtenir une émulsion d'une stabilité remarquable.

Des émulsions à base de cyclodextrines (monomères) et de composés lipophiles en milieu aqueux ont déjà été décrites. A ce sujet, il est en particulier connu que si on met en présence en milieu aqueux des cyclodextrines et un composé lipophile, on observe la formation de complexes d'inclusions entre les parties lipophiles dudit composé et les cyclodextrines.

Toutefois, jusqu'à présent, ces complexes d'inclusions ont toujours été décrits comme ayant un caractère cristallin, ce qui facilite la précipitation du complexe et résulte en une émulsion de stabilité insuffisante pour des applications à l'échelle industrielle, comme dans le domaine des cosmétiques, de la pharmacie et de l'agro-alimentaire.

Des solutions aqueuses de composé lipophile contenant des oligomères de cyclodextrine ont également été rapportées. Par exemple, le document JP 61/227517 décrit de telles solutions, à base de trimères ou tétramères de poly-beta-cyclodextrine (c'est-à-dire, des composés cyclodextrine de faible poids moléculaire et donc très solubles dans l'eau). Cependant, ce document n'envisage pas la préparation d'émulsion. Par ailleurs, les inventeurs ont démontré que de tels oligomères ne sont pas utilisables pour former des émulsions simples ou multiples huile-dans-l'eau et eau-dans-l'huile, et que seuls des polymères de cyclodextrine sont efficaces pour préparer de telles émulsions. Les inventeurs ont par ailleurs démontré de manière surprenante que des polymères à base d'unités cyclodextrine (e.g., poly α-, β- et/ou γ-cyclodextrines) ou des polymères hydrophiles portant des cyclodextrines (e.g., polysaccharides sur lesquels sont greffées des cyclodextrines) étaient non seulement très efficaces pour former de telles émulsions, mais aussi que celles-ci avaient une stabilité remarquable.

La présente invention repose sur la découverte toute à fait inattendue de la possibilité de former des complexes d'inclusion entres des polymères à base d'unités cyclodextrine (e.g., poly α-, β- et/ou γ-cyclodextrines) ou des polymères hydrophiles portant des cyclodextrines (e.g., polysaccharides sur lesquels sont greffées des cyclodextrines) et des composés lipophiles, lesquels complexes peuvent remplir la fonction de « tensioactifs » en tant que tels en raison de leur caractère amphiphile, et peuvent stabiliser de façon remarquable des émulsions.

Ainsi, un aspect de l'invention est de fournir des compositions comprenant une émulsion exempte de toutes traces de tensioactifs, pouvant avantageusement se substituer aux compositions connues de l'état de la technique pour la vectorisation de principes actifs ou de produits cosmétiques. Dans ce cadre, un des objets de l'invention repose sur l'encapsulation de composés lipophiles d'intérêt pour la préparation de compositions ayant au moins l'une des propriétés suivantes :
- Atténuation, voire masquage, de l'odeur d'un composé lipophile composant l'émulsion (e.g., l'huile essentielle de lavandin),
- Atténuation, voire masquage du mauvais goût d'un composé lipophile composant l'émulsion (e.g., l'huile de poisson),
- amélioration de la stabilité pour des composés lipophiles facilement oxydables (e.g., l'huile de bourrache),
- conservation de la stabilité pour des composés lipophiles facilement volatiles (e.g., ester beta-alanine, parfum),
- augmentation de la solubilité pour des composés lipophiles insolubles (e.g ., huile de gingembre, géraniol).

L'invention a également pour but de fournir des émulsions exemptes de tensioactifs et présentant néanmoins une stabilité suffisante pour pouvoir être stockées pendant une durée au moins de l'ordre de quelques semaines, voire quelques mois.

Ainsi, selon un aspect de l'invention, il est proposé une émulsion simple ou multiple **comprenant** un complexe d'inclusion non-covalent et non-cristallin formé (i) d'un polymère **I** à base d'unités cyclodextrine ou d'un polymère hydrophile **II** portant des cyclodextrines et (ii) d'un composé lipophile, **dans laquelle le polymère à base d'unités cyclodextrine et le polymère hydrophile portant des cyclodextrines contiennent au moins 10 unités cyclodextrine.**

Avantageusement, le polymère à base d'unités cyclodextrine et le polymère hydrophile portant des cyclodextrines contiennent au moins 10 unités cyclodextrine, de préférence au moins 15 unités cyclodextrines, et avantageusement au moins 20 unités cyclodextrines. De façon particulièrement avantageuse, on préfère que les polymère à base d'unités cyclodextrine et polymères hydrophiles portant des cyclodextrines comprennent en moyenne au moins 100 unités cyclodextrines, de préférence au moins 200 unités cyclodextrines, et avantageusement au moins 300 unités cyclodextrines. Typiquement, les polymères à base d'unités cyclodextrine et polymères hydrophiles portant des cyclodextrines comprennent en moyenne au moins 400 unités cyclodextrines.

Avantageusement, le polymère à base d'unités cyclodextrine et le polymère hydrophile portant des cyclodextrines contiennent en moyenne entre 10 et 1500 unités cyclodextrine au sein de leur structure, de préférence en moyenne entre 10 et 1000 unités cyclodextrine, de préférence en moyenne entre 15 et 800 unités cyclodextrine, de préférence en moyenne entre 50 et 600 unités cyclodextrine, et avantageusement en moyenne entre 100 et 400 unités cyclodextrine.

Selon certains modes de réalisation, le polymère à base d'unités cyclodextrine et le polymère hydrophile portant des cyclodextrines utilisés dans le contexte de la présente invention ont une masse molaire plus élevée. Par exemple, le polymère à base d'unités cyclodextrine et le polymère hydrophile portant des cyclodextrines peuvent comporter en moyenne entre 10 et 2000 unités cyclodextrine au sein de leur structure, de préférence en moyenne entre 100 et 1800 unités cyclodextrine, de préférence en moyenne entre 500 et 1600 unités cyclodextrine, et avantageusement en moyenne entre 800 et 1500 unités cyclodextrine.

Par « polymère hydrophile portant des cyclodextrines », on entend dans la présente un polymère hydrophile sur lequel des monomères cyclodextrine sont greffés. Autrement dit, il s'agit d'un polymère hydrophile sur lequel des monomères cyclodextrine sont attachés par une liaison covalente. Ces monomères cyclodextrine peuvent être identiques ou différents sur un même polymère hydrophile.

Le polymère hydrophile peut être tout polymère hydrophile neutre, cationique ou anionique. Par exemple, il peut s'agir d'un polymère hydrophile couramment utilisé dans la formulation de compositions pharmaceutiques, cosmétiques et/ou agro-alimentaires. Par exemple, les dispersions aqueuses de polymère sont couramment utilisées dans l'industrie pharmaceutique pour pelliculer les formes galéniques destinées à la voie orale et permettre une libération contrôlée du principe actif. Les polymères hydrophiles sont de plus en plus utilisés pour leur capacité à retenir l'eau, leur caractère filmogène et leur maintient à la surface de la peau sans pouvoir de pénétration. On peut citer à titre d'exemple les polysaccharides (tels que l'alginate de sodium, l'alginate de propylène glycol, les galactomannanes (gel d'aloès, gomme de guar), la gomme xanthane (Rhodopol®), les dérivés de la cellulose (comme l'hydroxyéthylcellulose, l'hydroxypropylcellulose, l'hydroxypropylméthylcellulose, la méthylcellulose, ou la carboxyméthylcellulose); les polymères acryliques et vinyliques (comme les carbomères ou Carbopols®, Acrysols®, les polymères cyanoacryliques, la polyvinylpyrrolidone ou povidone) ; les alcools polyvinyliques ; les polyéthylènes glycols ; ou les polyquaterniums.

Les polymères de silicone (comme le poly(methylhydrosiloxane) ou le poly(methylhydrosiloxane-co-dimethylsiloxane)) ne sont pas considérés comme des polymères hydrophiles II selon la présente invention.

L'émulsion peut être une émulsion huile-dans-l'eau ou eau-dans-l'huile, simple ou multiple. De préférence, il s'agit d'une émulsion simple huile-dans-l'eau.

Le polymère **I** ou **II** peut être choisi dans le groupe comprenant:
- les poly-α, poly-β ou poly-γ-cyclodextrines,
- les copolymères de α, β et/ou γ-cyclodextrines,
- les polymères naturels ou synthétiques sur lesquels sont greffées des α, β et/ou γ-cyclodextrines,
- ou un mélange de ceux-ci ;
dans lesquels les unités α, β et/ou γ-cyclodextrine sont éventuellement modifiées.

En particulier, le polymère **I** ou **II** peut être choisi dans le groupe comprenant:
- les poly-β-cyclodextrines,
- les poly-γ-cyclodextrines,
- les polysaccharides sur lesquels sont greffées des β-cyclodextrines et/ou γ-cyclodextrines,
- ou un mélange de ceux-ci.

Les polymères I à base d'unités cyclodextrines comportent en moyenne au moins 10 unités cyclodextrines, de préférence au moins 15 unités cyclodextrines, et avantageusement au moins 20 unités cyclodextrines. De façon particulièrement avantageuse, on préfère que les polymères à base d'unités cyclodextrines comprennent en moyenne au moins 100 unités cyclodextrines, de préférence au moins 200 unités cyclodextrines, et avantageusement au moins 300 unités cyclodextrines. Typiquement, les polymères à base d'unités cyclodextrines comprennent en moyenne au moins 400 unités cyclodextrines.

Le nombre moyen d'unités cyclodextrines présentes dans les polymères d'une émulsion de l'invention peut par exemple être établi par chromatographie d'exclusion stérique et par résonance magnétique nucléaire.

En général, le polymère **I** à base d'unités cyclodextrine comporte en moyenne entre 10 et 1500 unités cyclodextrine au sein de sa structure, de préférence en moyenne entre 10 et 1000 unités cyclodextrine, de préférence en moyenne entre 15 et 800 unités cyclodextrine, de préférence en moyenne entre 50 et 600 unités cyclodextrine, et avantageusement en moyenne entre 100 et 400 unités cyclodextrine.

Selon certains modes de réalisation, les polymères **I** utilisés dans le contexte de la présente invention ont une masse molaire plus élevée. Par exemple, le polymère **I** à base d'unités cyclodextrine peut comporter en moyenne entre 10 et 2000 unités cyclodextrine au sein de sa structure, de préférence en moyenne entre 100 et 1800 unités cyclodextrine, de préférence en moyenne entre 500 et 1600 unités cyclodextrine, et avantageusement en moyenne entre 800 et 1500 unités cyclodextrine.

Les unités cyclodextrines présentes au sein des polymères **I** ou **II** peuvent, de façon générale, être des α-cyclodextrines, des β-cyclodextrines, des γ-cyclodextrines, ou bien des mélanges d'au moins deux de ces types de cyclodextrines. Avantageusement, les unités cyclodextrines présentes dans les polymères **I** ou **II** comprennent de préférence des β-cyclodextrines et/ou γ-cyclodextrines. Selon une variante particulière, toutes les unités cyclodextrines présentes dans les polymères **I** ou **II** sont des β-cyclodextrines. Selon une autre variante particulière, toutes les unités cyclodextrines présentes dans les polymères **I** ou **II** sont des γ-cyclodextrines.

Au sein des polymères **I** à base d'unités cyclodextrine , les unités cyclodextrine sont généralement liées entre elles par des chaînes hydrocarbonées de 3 à 50 atomes de carbone, linéaires ou ramifiées, éventuellement interrompues par un ou plusieurs atomes d'oxygène, et ces chaînes étant de préférence des chaînes alkyles, alcényles, ou alcynyles de 3 à 50 atomes de carbone, ou bien encore des chaînes polyéthers de 3 à 50 atomes de carbone, ces chaînes pouvant être substituées par des groupements hydrophiles (groupements hydroxy par exemple). Les chaînes liant entre elles les unités cyclodextrines comportent au moins 3 atomes de carbone et de préférence de 4 à 50 atomes de carbone, le chemin le plus court entre deux unités cyclodextrines étant de préférence constitué par une chaîne comportant entre 3 et 8 atomes de carbone.

Avantageusement, les chaînes hydrocarbonées liant entre elles deux unités cyclodextrines au sein d'un polymère **I** à base d'unités cyclodextrine répondent à la formule générale un groupement de formule -O-(CH₂-CHOR¹-CH₂)ₙ-O-. où n est un entier compris entre 1 et 50 (généralement compris entre 2 et 10) et où, dans chacune des n unités (CH₂-CHOR¹-CH₂), R¹ désigne soit un atome d'hydrogène, soit une chaîne -CH₂-CHOH-CH₂-O- reliée à une unité cyclodextrine du polymère.

Ainsi, les polymères **I** peuvent typiquement être obtenus par une réticulation de molécules de cyclodextrines avec des composés bifonctionnels capables de former des liaisons covalentes avec les groupements hydroxyles des cyclodextrines. Par exemple, il peut s'agir d'acides bicarboxyliques tels que l'acide citrique, l'acide sébacique, l'acide fumarique, l'acide glutamique, l'acide maléique, l'acide malique, l'acide malonique, l'acide oxalique, l'acide succinique, l'acide glutarique, l'acide téréphtalique, l'acide isophtalique, l'acide oxaloacétique, l'acide phtalique, l'acide adipique ou l'acide butanedioïque.

Par exemple, les polymères **I** peuvent être obtenus par polycondensation de molécules de cyclodextrines et d'épichlorhydrine, généralement en milieu basique (généralement dans un milieu aqueux additionné de soude, à une concentration massique de 10 à 40%), le ratio molaire cyclodextrines/épichlorhydrine étant de préférence compris entre 1 : 15 et 1 : 1, et avantageusement entre 1 : 15 et 1 : 8. Pour plus de détails concernant cette synthèse et contrôle du nombre moyen d'unités cyclodextrines intégrées au sein des polymères à base d'unités cyclodextrine obtenus selon ce procédé, on pourra notamment se référer aux articles suivants :
- E. Renard et al., European Polymer Journal, vol. 33, No 1, pp 49-57 (1997) **[9]**
- Gref et al., International Journal of Pharmaceutics, Vol. 332, Issues 1-2, Pages 185-191 (2007) **[10]**
- Gref et al., J. Control Release, 111(3):316-24 (2006) **[11]**
- Gref et al., Journal of colloid and interface science, 307(1):83-93 (2007) **[12]**
- Blanchemain et al., Acta Biomaterialia, Volume 4, Issue 6, November 2008, Pages 1725-1733 **[13]**

Les polymères I peuvent également être obtenus par polycondensation de molécules de cyclodextrines et d'hexaméthylène diisocyanate, comme décrit par exemple dans Elif Yilmaz Ozmen et al. Bioresource Technology, Volume 99, Issue 3, Pages 526-531 (2008) **[14].**

Les polymères **I** peuvent également être obtenus par polycondensation de molécules de cyclodextrines et un polyéthylène glycole fonctionnalisé, comme décrit par exemple dans :
- Cesteros et al., European Polymer Journal, Volume 45, Issue 3, Pages 674-679 (2009) (PEG acylé) **[15]**
- Salmaso et al., International Journal of Pharmaceutics, Volume 345, Issues 1-2, Pages 42-50 (2007) (PEG diaminé) **[16]**

Les polymères **I** peuvent également être obtenus par polycondensation de molécules de cyclodextrines et plusieurs branches oligoethylimine, pour former un polymère étoilé, comme décrit par exemple dans Yang et al., Biomaterials, Volume 28, Issue 21, Pages 3245-3254 (2007). **[17]**

Quelle que soit la nature exacte des chaînes hydrocarbonées liant entre elles les unités cyclodextrines, en général, la masse totale des motifs cyclodextrines présents au sein des polymères **I** représente au moins 30%, avantageusement au moins 40 %, et encore plus préférentiellement au moins 50%, de la masse totale des desdits polymères, cette masse totale des motifs cyclodextrine représentant généralement entre 30 et 80%, et de préférence entre 40 et 75% de la masse totale des polymères à base d'unités cyclodextrine.

Ce pourcentage massique de cyclodextrines dans les polymères utilisables dans le contexte de la présente invention peut par exemple être déterminé par résonance magnétique nucléaire (RMN).

Par ailleurs, le polymère **I** présent dans une émulsion selon l'invention possède en général une masse molaire moyenne en nombre comprise entre 10 000 et 3 000 000 g/mole, avantageusement entre 20 000 et 2 000 000, et de préférence entre 100 000 et 1 500 000 g/mole.

Avantageusement, le polymère **I** présent dans une émulsion selon l'invention possède une masse molaire moyenne en nombre supérieure ou égale à 100 000 g/mole. Ceci permet d'arriver à des émulsions d'une stabilité particulièrement élevée.

Avantageusement, le polymère **I** peut posséder un indice de polydispersité (c'est à dire un rapport de la masse molaire moyenne en poids sur la masse molaire moyenne en nombre) le moins élevé possible, de préférence inférieur à 3, et encore plus avantageusement inférieur à 2.

Dans certains modes de réalisation, le polymère **I** peut être un poly-β-cyclodextrine répondant à la Formule **I** suivante : 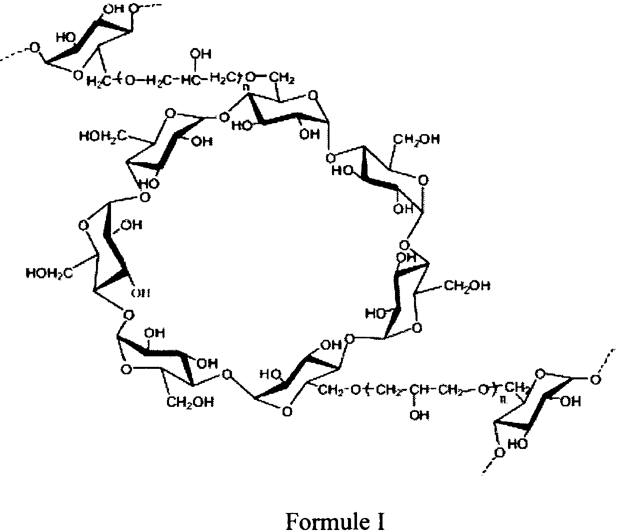 dans laquelle n représente un entier compris entre 1 et 50, de préférence entre 2 et 10 ; et le nombre d'unités beta-cyclodextrine est en moyenne compris entre 10 et 1500, de préférence en moyenne entre 10 et 1000, de préférence en moyenne entre 15 et 800, de préférence en moyenne entre 50 et 600, et avantageusement en moyenne entre 100 et 400 unités.

Dans la structure ci-dessus, les monosaccharides aux extrémités de la structure (i.e., avec des liaisons représentées en pointillés) schématisent la continuité du polymère (i.e., enchainement d'unités β-cyclodextrines formant le reste du polymère).

Dans certains modes de réalisation, le polymère **I** peut être un poly-β-cyclodextrine de masse molaire plus élevée, et peut répondre à la formule I ci-dessus, dans laquelle n représente un entier compris entre 1 et 50, de préférence entre 2 et 10 ; et le nombre d'unités beta-cyclodextrine est en moyenne compris entre 10 et 2000, de préférence en moyenne entre 100 et 1800 unités, de préférence en moyenne entre 500 et 1600 unités, et avantageusement en moyenne entre 800 et 1500 unités.

Dans certains modes de réalisation, le polymère **I** peut être un poly-α-cyclodextrine répondant à la Formule I ci-dessus dans laquelle les unités β-cyclodextrines sont remplacées par des α-cyclodextrines.

Dans certains modes de réalisation, le polymère **I** peut être un poly-γ-cyclodextrine répondant à la Formule **I** ci-dessus dans laquelle les unités β-cyclodextrines sont remplacées par des γ-cyclodextrines.

Le polymère **II** peut être un polymère synthétique ou naturel généralement hydrophile. Il peut s'agir par exemple d'un polysaccharide. Par exemple, il peut s'agir de l'acide hyaluronique , l'acide alginique, le chitosane, la chitine, le scleroglucane, le dextrane, l'amilose, l'amilopectine, un dérivé de la cellulose, l'amidon, le pullulane, la pectine, un alginate, l'héparine, l'ulvane, un caragheenane, le fucane, le curdlan, le xylane, l'acide polyguluronique, le xanthane, l'arabinane, ou l'acide polymannuronique. Avantageusement, le polymère hydrophile **II** peut être l'acide hyaluronique. En effet, celui-ci peut être intéressant, notamment pour des émulsions à des fins cosmétiques, en raison de ses propriétés de bioadhésion. Ainsi, une composition comprenant une émulsion selon l'invention à base d'acide hyaluronique portant des cyclodextrines pourra permettre de renforcer, ou en tout cas de prolonger, l'effet cosmétique recherché, car les gouttelettes de l'émulsion auront tendance à mieux rester en surface de la peau (en raison des propriétés bioadhésives de l'acide hyaluronique).

Le polymère hydrophile **II** peut être éventuellement modifié, c'est-à-dire que certains de ses groupements peuvent avoir subi une transformation chimique. Par exemple, le polymère hydrophile **II** peut être un polysaccharide sulfaté naturel comme le chondroitin sulfate, le fucoïdane ou l'héparane sulfate. L'héparane sulfate est un glycane endogène bioactif chez l'homme ; le fucoïdane est aussi un polysaccharide sulfaté mais d'origine marine et extrait d'algues brunes. Le fucoïdane présente des activités sur les systèmes biologiques mammifères qui pourraient en faire un substitut aux polysaccharides sulfatés d'origine animale utilisés en thérapeutique (héparine). Le fucoïdane compte parmi les inhibiteurs les plus puissants du système du Complément, qui occupe une place majeure dans les mécanismes de défense de l'organisme. Activé en début de toute agression tissulaire, le Complément constitue un mécanisme de défense précoce au cours de la réaction immunitaire. Cependant l'activation incontrôlée du Complément peut être à l'origine de dommages tissulaires, comme lors de rejet de transplantation, de pathologies inflammatoires et de certaines pathologies neurodégénératives comme la maladie d'Alzheimer. Le fucoïdane bloque le complément en inhibant les deux voies d'activation classique et alterne du système du Complément.

Alternativement, des polysaccharides naturels peuvent être sulfatés. Ceci peut être réalisé par exemple par transformation enzymatique (WO 2006/124801). **[18]** Alternativement ces polysaccharides peuvent être extraits de sources naturelles, telles que certaines algues comme la *Durlillaea antartica* ou des algues vertes de la famille des Ulves (*Ulva* et *Enteromorpha sp*) ayant des propriétés bénéfiques intrinsèques (cicatrisation de la peau) Alternativement, le polysaccharide peut contenir à l'état naturel des groupements sulfate. On peut citer par exemple l'héparine et l'ulvane.

Des méthodes pour greffer des cyclodextrines à des polymères naturels ou synthétiques, en particulier des polysaccharides, sont connues. Par exemple, l'homme du métier pourra s'inspirer des méthodes décrites dans :
β-cyclodextrines greffées sur du chitosan :
   - Prabaharan et al., International Journal of Biological Macromolecules, Volume 44, Issue 4, Pages 320-325(2009*)* **[19]**
   - Zhang et al., "Chitosan bearing pendant cyclodextrin as a carrier for controlled protein release ", Carbohydrate Polymers, In Press, Corrected Proof, Available online 30 January 2009 *[20]*

Il est entendu que le polymère hydrophile II portant des cyclodextrines peut être chimiquement modifié en utilisant des techniques synthétiques classiques connues de l'homme du métier. On pourra se référer par exemple à :
- Prabaharan et al., Carbohydrate Polymers, Volume 73, Issue 1, Pages 117-125 (2008) **[21]**
- Prabaharan et al., Carbohydrate Polymers, Volume 73, Issue 1, Pages 117-125 (2008) **[22]**
qui décrivent la fonctionnalisation d'un carboxyméthyl chitosan-β-cyclodextrine avec l'ester de méthyle de cystéine, conduisant à une version sulfurée (-SH) du polymère ayant des propriétés mucoadhésives améliorées.

On peut également envisager un co-polymère à base d'unités cyclodextrine et d'un autre monomère, comme décrit par exemple dans Lu et al., European Polymer Journal, Volume 44, Issue 7, Pages 2140-2145 (2008) (copolymérisation radicalaire de polylactic acide et de dérivés vinyles de β-cyclodextrines. **[23]**

On peut également envisager un polymère à base d'unités cyclodextrine (polymère I) greffé sur un autre polymère, comme décrit par exemple dans Blanchemain et al., Acta Biomaterialia, Volume 4, Issue 5, Pages 1392-1400 (2008), **[24]**ou réticulé avec un polysaccharide, comme décrit par exemple dans Zha et al., Journal of Membrane Science, Volume 321, Issue 2, Pages 316-323 (2008). **[25]**

Les émulsions selon l'invention, qui intègrent des polymères hydrophiles II à base de polysaccharides greffés, sont particulièrement intéressantes en termes de bioadhésivité, ce qui les rend extrêmement avantageuses pour une application par exemple, sur des muqueuses. Ainsi, pour une application par voie oculaire, il s'avère particulièrement intéressant que les polymères hydrophiles II présents dans l'émulsion soient des acides hyaluroniques greffés avec des cyclodextrines.

Par ailleurs, sans vouloir être lié en aucune façon à une théorie particulière, il semble pouvoir être avancé que, dans la mesure où les polymères hydrophiles II sont des polysaccharides porteurs de cyclodextrines, la structure des gouttelettes de la phase dispersée est en règle générale telle que la couche externe des gouttelettes est essentiellement constituée de polysaccharides. En tout état de cause, en particulier lorsque les polysaccharides sont des dextranes, les gouttelettes présentent en général tendance à adhérer à la surface de certaines muqueuses, au niveau desquelles elles délivrent ensuite, généralement de façon progressive, le composé lipophile qu'elles contiennent. On peut ainsi, en appliquant une composition selon l'invention comprenant un composé lipophile à effet thérapeutique sur une muqueuse donnée (muqueuse nasale, oculaire...) réaliser une administration sélective du composé au niveau de cette muqueuse. Par ailleurs, il semble également pouvoir être avancé que, lors d'une administration par voie orale, la structure spécifique des gouttelettes favorise leur translocation à travers l'épithélium digestif, et le passage des principes actifs lipophiles encapsulés dans le réseau sanguin.

Dans le cas où les émulsions selon l'invention sont destinées à la vectorisation de composés de type médicaments, il est en général avantageux que les polymères I et /ou II soient substitués par des groupements permettant un ciblage cellulaire. Dans ce cadre, il peut être intéressant par exemple que les polymères I et /ou II soient complexés par des ligands de type acide folique.

Par ailleurs, de façon à améliorer le caractère "furtif" des émulsions selon l'invention (c'est-à-dire leur capacité à circuler de façon prolongée dans l'organisme tout en évitant une détection par le système immunitaire), il peut être avantageux que les gouttelettes formant les émulsions de l'invention présentent des groupements externes de type polyéthylèneglycol (PEG). Pour ce faire, on peut par exemple mettre en oeuvre à titre de polymère II des macromolécules de polysaccharides (de préférence des fucoïdanes, l'héparine ou l'acide hyaluronique) éventuellement porteuses à la fois de cyclodextrines tels que définis précédemment, et de chaînes PEG.

Un autre mode de réalisation de particules greffées par des groupements PEG consiste à additionner au système associatif des polymères I et/ou II et du composé lipophile, des composés de type PEG- [Alk] où Alk représente un groupement alkyle en C10 à C18, de préférence de C12 à C16, ou bien un groupement adamantyle. Le cas échéant, l'addition des composés de type PEG- [Alk] peut être effectuée avant ou après formation de l'émulsion à partir du système associatif (polymère I/II + composé lipophile). En général, on préfère toutefois que cette addition soit réalisée après formation des particules.

Quelle que soit la nature du polymère I ou II, les cyclodextrines formant le polymère (I) ou présentes sur le polymère (II) peuvent être modifiées, c'est-à-dire que certains de leurs groupements peuvent avoir subi une transformation chimique. Par exemple, il peut s'agir de cyclodextrines portant des groupements sulfate, alkyle, hydroxyalkyle (e.g., hydroxyéthyle, hydroxypropyle, dihydroxypropyle, hydroxybutyle, hydroxyisobutyle), carboxyakyle (e.g., carboxyméthyle), glycosyle et/ou amino.

Cela étant, compte tenu de la présence de motifs cyclodextrines au sein de leur structure, les polymères I et II sont particulièrement adaptés pour réaliser l'encapsulation de composés chimiques, et tout particulièrement pour réaliser l'encapsulation de composés chimiques présentant des groupements de nature hydrophobe. Ainsi, les polymères I et II selon l'invention sont à même d'intégrer, sous forme encapsulée, de nombreux types de composés chimiques neutres ou chargés. Les composés susceptibles de pouvoir être encapsulés au sein de ces polymères comprennent en particulier les composés présentant des groupement hydrophobes, notamment des groupements alkyles, comportant en général de 6 à 18 atomes de carbone.

De façon plus spécifique, les composés lipophiles susceptibles d'être utilisés dans les émulsions selon l'invention sont tous composés susceptibles de former des complexes d'inclusion avec les unités cyclodextrines que les polymères I et II comprennent. Pour plus de détails en ce qui concerne la formation de complexes d'inclusion entre des composés chimiques et des cyclodextrines, ainsi que sur la nature des composés susceptibles de former de tels complexes, on pourra notamment se reporter à "Cyclodextrins and their inclusion complexes", Szejtli J., Academia Kiado, Budapest, 1982. **[26]**

De façon générale, une simple mise en contact des composés lipophiles précités avec un polymère I ou II selon l'invention suffit pour réaliser l'encapsulation desdits composés lipophiles par lesdits polymères, en particulier lorsque ladite composition est essentiellement à base d'eau et des polymères I ou II.

Le composé lipophile est en général un composé présentant des groupements de nature hydrophobe, avantageusement des groupements de type chaînes hydrocarbonées comportant de 8 à 18 atomes de carbone, et de préférence de 10 à 18 atomes de carbone. De façon avantageuse, ce composé lipophile est un composé susceptible de former un complexe d'inclusion avec l'une des unités cyclodextrines comprises dans les polymères I ou II.

Le composé lipophile des émulsions selon l'invention peut être un composé choisi dans le groupe comprenant:
- les corps gras choisis dans le groupe comprenant les huiles naturelles d'origine végétale, animale ou marine (telles que l'huile d'olive, l'huile de sésame, l'huile d'argan, l'huile de palme, l'huile de soja, l'huile de pastel, l'huile de tortue, l'huile de babassu, l'aloe vera, l'huile d'avocat, l'allantoïne, le bisabol, l'huile de pépins de raisin, l'huile d'abricot, l'huile de germe de blé, l'huile d'amande, l'huile d'arachide, l'huile de noix de macadamia, l'huile d'argousier, l'huile d'onagre, l'huile de bourrache, l'huile de gingembre, le géraniol, l'huile de jujube, l'huile de vison, la lanoline), les huiles synthétiques, les huiles minérales (telles que l'isohexadécane, l'isoparaffin, la ceresin, la vaseline), les huiles hydrogénées, l'huile de silicone, les composés hydrocarbonés (tels que la paraffine liquide, les terpènes, le squalène), les acides gras saturés ou insaturés (tels que l'acide myristique), les esters d'acide gras, les cires (comme la cire de baleine, la cire d'abeille, l'huile de jojoba qui est en fait une cire liquide), les alcools gras (tels que l'alcool myristylique, l'alcool cétylique, l'alcool stéarylique, l'alcool myricylique), les beurres (comme le beurre de karité ou le beurre de cacao), les cire-esters, ou un mélange de ceux-ci,
- les composés odorants lipophiles utilisés dans la fabrication des parfums, et
- les principes actifs lipophiles.

Les corps gras peuvent être choisis parmi tout corps gras couramment utilisés dans la formulation de compositions pharmaceutiques, cosmétiques et/ou agro-alimentaires. Les exemples cités ci-dessus ne sont qu'une illustration des corps gras susceptibles d'être utilisés, et ne sont en aucun cas limitants.

Dans la présente, on entend par « principe actif » une molécule qui possède un effet thérapeutique ou cosmétique. Par exemple, il peut s'agir de toute molécule ayant des propriétés thérapeutiques entrant dans la composition d'un médicament. On pourra citer par exemple les anti-inflammatoires non stéroïdiens (AINS), les Abortifs, les Alpha-bloquants, les Alpha2-agonistes, les Aminosides, les Analgésiques, les Anesthésiques, les Anesthésiques locaux, les Anorexigènes, les Antagonistes 5HT3, les Antagonistes du calcium, les Antiangoreux, les Antiarythmiques, les Antibiotiques, les Anticholinergiques, les Anticholinestérasiques, les Antidiabétiques, les Antidiarrhéiques, les Antidépresseurs, les Antihistaminiques, les Antihypertenseurs, les Antimycosiques, les Antipaludéens, les Antiparasitaires, les Antipsychotiques, les Antipyrétiques, les Antirétroviraux, les Antiseptiques, les Antispasmodiques, les Antiviraux, les Antiémétiques, les Antiépileptiques, les Anxiolytiques, les Barbituriques, les Benzodiazépines, les Bronchodilatateurs, les Beta-bloquants, les Agents chimiothérapeutiques, les Corticostéroïdes, les Diurétiques, les Diurétiques de l'anse, les Diurétiques osmotique, les Dépresseurs, les Glucocorticoïdes, les Hallucinogènes, les Hypnotiques, les Immunosuppresseurs, les Inhibiteurs de l'anhydrase carbonique, les Inhibiteurs de la neuraminidase, les Inhibiteurs de la pompe à protons, les Inhibiteurs du TNF, les Inhibiteurs sélectifs de la recapture de la sérotonine, les inhibiteurs de la HMG-CoA réductase (ou statines), les Kératolytiques, les Laxatifs, les minéralocorticoïdes, les Myorelaxants, les Neuroleptiques, les Psychotropes, les Spasmolytiques, les Stimulants, les Sédatifs, les Tocolytiques ou les Vasodilatateurs. Cette liste n'est pas exhaustive et s'étend à tout principe actif thérapeutique connu de l'homme du métier.

Il peut s'agir également de toute molécule entrant dans la composition d'une préparation cosmétique qui assure l'efficacité du produit (par opposition aux autres ingrédients de la composition tels que les excipients ou autres additifs (adjuvants (pour parfumer, faire mousser, etc.), conservateurs notamment parabènes, colorants, antioxydants, émulsifiants, stabilisateurs de pH, tensioactifs, agents de contrôle de la viscosité, etc.) qui assurent une fonction différente). Le terme principe actif est couramment utilisé en cosmétique même si l'expression principe actif est normalement réservée aux médicaments. Dans la présente description, le terme « principe actif » est utilisé pour désigner une substance active à effet thérapeutique ou cosmétique. Ainsi, un principe actif peut être toute molécule assurant l'effet cosmétique des produits cosmétiques tels que les produits d'hygiène (démaquillant, dentifrice, déodorant, gel douche, gel nettoyant intime, savon, shampoing), les produits de soin (crème antirides, crème de jour, crème de nuit, crème hydratante, eau florale, gommage, lait, masque de beauté, baume pour les lèvres, tonique), les produits capillaires (après-shampoing, défrisant, gel, huile, laque, masque, teinture), les produits de maquillage (anti-cerne, autobronzant, ligneur (eyeliner), fard, fond de teint, khôl, mascara, poudre, produit pour blanchir la peau, rouge à lèvres, vernis à ongles), les parfums (eau de Cologne, eau de toilette, parfum), les produits solaires (crèmes, huiles ou lotions après-soleil et solaires), les produits pour le rasage et les produits dépilatoires (après-rasage, crème dépilatoire, mousse à raser), les préparations pour bains et douches (bain moussant, huile de bain, sels de bain), pour en citer quelque uns. Les cosmétiques sont des produits d'hygiène et d'embellissement. Un cosmétique est une substance ou une préparation destinée à être mise en contact avec diverses parties superficielles du corps humain, notamment l'épiderme, les systèmes pileux et capillaires, les organes externes, les dents et les muqueuses, en vue, exclusivement ou principalement, de les nettoyer, protéger, parfumer, maintenir en bon état le corps humain, de modifier son aspect ou d'en corriger l'odeur. Ainsi, par « effet cosmétique », on entend dans la présente description l'effet d'hygiène ou d'embellissement précité que le produit cosmétique est destiné à, et conçu pour, accomplir.

A titre de principes actifs cosmétiques, on pourra citer par exemple les acides de fruits (exfoliants), le rétinol ou vitamine A (antioxydant), certaines huiles essentielles, l'acide hyaluronique (hydratant), les huiles végétales telles que l'huile de jojoba (simmondsia chinensis) qui est par ailleurs un léger filtre solaire naturel, l'huile d'avocat (persea gratissima), l'huile d'amande (prunus dulcis), l'huile d'olive (olea europaea), l'huile de germe de blé (triticum vulgare), l'huile d'arachide, l'huile de noix de macadamia (macadamia ternifolia), l'huile de palme, l'huile d'argousier (hippophae rhamnoides), l'huile de bourrache, l'huile d'onagre, l'huile de pépins de raisin (vitis vinifera), le beurre de cacao (theobroma cacao), le beurre de karité (butyrospermum parkii), et les substances hydratantes telle que les protéines de soie (qui sont des substances actives provenant de cocons de vers séricigènes), l'aloe vera, les extraits d'algues, et les acides aminés (qui sont responsables du métabolisme cellulaire, fixent bien l'eau et participent à la formation de l'agent d'hydratation cutanée (NMF)). Cette liste n'est pas exhaustive et s'étend à tout principe actif cosmétique connu de l'homme du métier.

La nature exacte du composé lipophile peut varier en une assez large mesure. Toutefois, notamment dans la mesure où les polymères I ou II peuvent être choisis parmi des composés non toxiques et biocompatibles, et que la présence de traces de solvants organiques ou de tensioactifs peut être évitée, une des applications principales envisageable pour une émulsion selon l'invention est la vectorisation de principes actifs, notamment des composés présentant un effet thérapeutique ou cosmétique. Ainsi, la présente invention concerne également une émulsion selon l'invention pour **son utilisation dans** la vectorisation de composés lipophiles présentant un effet thérapeutique ou cosmétique, ou à usage agroalimentaire (e.g., huile de poisson).

Ainsi, selon un mode de réalisation particulièrement avantageux, une émulsion selon l'invention peut comprendre à titre de composé lipophile au moins un composé actif à titre de médicament, ce composé lipophile actif à titre de médicament étant de préférence susceptible de former un complexe d'inclusion avec l'une des unités cyclodextrines comprises au sein des polymères I ou II.

Une telle émulsion selon l'invention est en général utilisable à titre de composition pharmaceutique pour une administration, par injection ou par voie orale, ou bien encore par voie dermique ou sous-cutanée, par voie nasale, par voie pulmonaire ou par voie oculaire et, de façon plus large, pour toute administration au niveau d'une muqueuse, ou au niveau d'un site précis (tumeur, lumière de certains vaisseaux sanguins...). Dans ce cadre, on préfère, le plus souvent, que la composition soit essentiellement constituée d'eau, des polymères I et/ou II et du composé lipophile, éventuellement en association avec un ou plusieurs excipient pharmacologiquement acceptables et adaptés à la voie d'administration envisagée. Toutefois, de façon large, une composition selon l'invention peut, dans ce type d'application, prendre la forme de toute formulation pharmaceutique intégrant une émulsion selon l'invention où le composé lipophile est un principe actif à titre de médicament. Dans le cas d'une composition spécifiquement destinée à une administration par injection intraveineuse, on préfère en général que les gouttelettes de la phase dispersée présentent un diamètre hydrodynamique moyen au plus égal à 200 nm.

Pour ce qui est des compositions destinées à une administration par injection intramusculaire, on préfère que les gouttelettes de la phase dispersée présentent un diamètre hydrodynamique moyen compris entre 200 et 5000 nm, de préférence inférieur à 1000 nm.

Par exemple, le composé lipophile à titre de principe actif peut être choisi dans le groupe comprenant les agents émollients, les agents anti- infectieux, les anticancéreux, les anti-inflammatoires, les anti-bactériens, les anti-fongiques, les anti-viraux, les anti-séborrhéiques, les anti-acnéiques, les anti-parasitaires, les opioïdes, les kératolytiques, les anti-histaminiques, les anesthésiques, les agents cicatrisants, les modificateurs de la pigmentation, les filtres solaires, les piégeurs de radicaux libres, les agents hydratants, les vitamines, des enzymes, ou encore des polypeptides.

Ainsi, à titre de composés lipophiles actifs à titre de médicaments utilisables dans les émulsions de l'invention, on peut notamment citer la molsidomine, le ketoconazole, le gliclazide, le diclofénac, le levonorgestrel, le paclitaxel, l'hydrocortisone, la pancratistatine, le kétoprofène, le diazépam, l'ibuprofène, la nifédipine, la testostérone, le tamoxifène, le furosémide, le tolbutamide, le chloramphénicol, la benzodiazépine, le naproxène, le déxaméthasone, le diflunisal, l'anadamide, la pilocarpine, la daunorubicine, la doxorubicine, la diazépame et le piroxicame.

Parmi les vitamines, on peut citer la vitamine A, la vitamine E, la vitamine D, la vitamine F, le beta-carotène, les carotènes.

Les compositions de l'invention comprenant un composé lipophile actif à titre de médicament induisent en général, suite à leur administration, une libération progressive du composé lipophile formant un complexe d'inclusion avec les cyclodextrines des polymères I ou II, en particulier lorsque ladite composition est administrée chez un patient par voie intraveineuse. On peut ainsi réaliser à l'aide d'une telle composition l'administration prolongée du composé lipophile actif.

Dans ce cadre, sans vouloir être lié en aucune façon à une théorie particulière, il semble pouvoir être avancé que la libération du composé lipophile s'effectue avec un équilibre de partage entre les gouttelettes de la phase dispersée et le milieu extérieur. Ainsi, il semble que la cavité hydrophobe interne de chaque cyclodextrine constitue un site potentiellement accepteur des molécules de principes actifs ou de leurs fragments lipophiles. Plus grande sera l'affinité du principe actif pour les cyclodextrines, plus sa libération sera ralentie. De ce fait, un composé lipophile actif à titre de médicament contenu dans une composition selon l'invention est le plus souvent libéré de façon préférentielle au niveau des cellules ou des tissus où ce composé est consommé, c'est-à-dire, le plus souvent, là où il joue un rôle thérapeutique effectif.

Toujours en ce qui concerne les compositions de l'invention comprenant un composé lipophile actif à titre de médicament, il est à noter que celles-ci sont en général administrables en tant que telles par voie orale.

Dans ce cas, elles peuvent permettre de réaliser l'administration par voie orale d'un composé lipophile actif de goût ou d'odeur désagréable (l'encapsulation est généralement à même de masquer ce goût ou cette odeur) ou bien encore d'un composé lipophile fragile et/ou difficilement absorbable par voie orale, comme par exemple un composé choisi parmi des anti-inflammatoires tels que le piroxicame, l'ibuprofène et le kétoprofène, des agents hypoglycémiques tels que le glicazide, des agents contraceptifs tels que le D-norgestrel, ou bien encore des composés antifongiques ou antiparasitaires tels que le kétoconazole ou l'albendazole.

Selon un autre mode de réalisation, les composés lipophiles présents au sein de des émulsions selon l'invention peuvent également être des actifs cosmétiques, et la composition selon l'invention peut alors être avantageusement utilisée à titre de composition cosmétique.

Dans ce cadre, les composés lipophiles peuvent par exemple être des composés odorants, par exemple de type terpènes, ou bien un mélange de tels composés (parfums, essences, huiles essentielles...). Dans une telle composition les composés odorants présentent en général un pouvoir irritant plus faible qu'à l'état non encapsulé, et ils sont libérés de façon retardée, ce qui améliore la tenue du parfum. De la même façon, d'autres types d'agents cosmétiques présentant de préférence un caractère hydrophobe, peuvent être immobilisés à titre de composé lipophile au sein d'une composition selon l'invention, puis libéré de façon progressive. Ainsi, une composition selon l'invention peut par exemple permettre la libération contrôlée d'agents antiperspirants ou bien encore antibactériens. Dans une composition selon l'invention destinée à un usage cosmétique, le composé lipophile peut également être un colorant irritant ou présentant une certaine toxicité, dont l'encapsulation permet de réduire les effets indésirables.

Selon un mode de réalisation, le composé lipophile peut être un agent anti-UV organique susceptible d'être utilisé dans les filtres solaires ou un systèmes photoprotecteurs capables de filtrer le rayonnement UV.

Selon l'invention, on entend désigner de manière générale par « système photoprotecteur capable de filtrer le rayonnement UV » ou « filtre solaire », tout composé ou toute association de composés qui, par certains mécanismes d'adsorption et ou réflexion et/ou diffusion du rayonnement UV-A ou UV-B, permet d'empêcher ou limiter la mise en contact du rayon rayonnement avec une surface (peau, cheveux) sur laquelle ce ou ces composés sont appliqués.

Les filtres photoprotecteurs sont typiquement des molécules synthétiques qui assurent une protection photochimique par absorption sélective de certains photons. Elles absorbent l'énergie des rayons ultraviolets. Il n'existe pas de filtre chimique efficace sur l'ensemble du spectre UV. Il existe des filtres à spectre d'absorption étroit, sélectifs des UVB (par exemple les benzimidazoles, benzylidène camphre, cinnamates, esters de l'acide para-amino-benzoïque PABA) et des filtres à spectre large qui sont efficaces dans l'UVA (par exemple les benzophénones, dérivés du dibenzoylméthane). Sur le plan qualitatif, pour obtenir le maximum d'absorption spectrale, plusieurs filtres sont habituellement associés. La concentration dans le produit fini ne dépasse généralement pas 6-8%. Sur le plan quantitatif, l'absorbance de chaque filtre est fonction de sa concentration.

Parmi les agents anti-UV organiques susceptibles d'être utilisés selon l'invention, on peut citer par exemple, la benzophénone-3, la benzophénone-4 , la benzophénone-8, le bis-éthylhexyloxyphénol méthoxyphényl triazine, le butyl méthoxydibenzoylméthane, le p-méthoxycinnamate de 2-éthoxyéthyle, le benzoate de diéthylamino hydroxybenzoyl hexyle, le drométrizole trisiloxane, le méthoxycinnamate d'éthylhexyle (méthoxycinnamate d'octyl ou « octinoxate »), le salicylate d'éthylhexyle, l'éthylhexyl triazone, le salicylate d'homométhyl, l'anthranilate de méthyle, le (4-)méthyl-benzylidène de camphre, le méthylène bis-benzotriazolyl tétraméthylbutylphénol, le cyanophénylcinnamate d'octyle, l'acide para-aminobenzoïque , l'acide phénylbenzimidazole sulfonique, le benzylidène malonate de polysiloxane, le salicylate de triéthanolamine et l'acide téréphtalylidène dicamphosulfonique.

Par exemple, le composé lipophile peut être un agent anti-UV organique, tel que le méthoxycinnamate d'éthylhexyle (aussi désigné par le terme « Parsol® MCX» dans la présente description) répondant à la structure suivante :

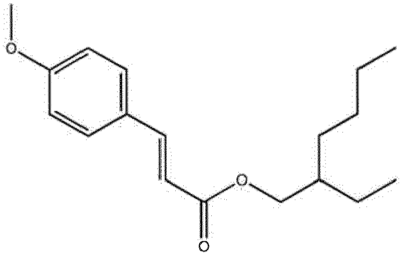

Dans ce cadre, un objet de la présente invention repose sur l'utilisation d'une émulsion telle que définie ci-dessus **dans laquelle l'émulsion comprend un agent anti-UV organique comme composé lipophile,** pour la fabrication de compositions pour la protection de la peau et/ou les cheveux contre le rayonnement ultraviolet, en particulier le rayonnement solaire. **Un** procédé de traitement cosmétique pour protéger la peau et/ou les cheveux contre le rayonnement ultraviolet, en particulier le rayonnement solaire, comprenant l'application sur la peau et/ou les cheveux d'une quantité efficace d'une composition comprenant une émulsion telle que définie ci-dessus **est décrit.**

**L'invention concerne également une composition comprenant une émulsion telle que définie ci-dessus pour son utilisation pour protéger la peau et/ou les cheveux contre le rayonnement ultraviolet, en particulier le rayonnement solaire.**

De façon plus générale, les compositions de l'invention peuvent être mises en oeuvre pour réaliser une libération progressive des composés lipophiles au sein d'un milieu dans lequel elles sont introduites ou bien pour limiter la mise en contact desdits composés lipophiles avec ledit milieu, par exemple en vue de leur protection, lorsqu'il s'agit de molécules fragiles vis-à-vis du milieu considéré, ou bien pour isoler des composés qui peuvent être des polluants (agents toxiques, irritants, réactifs...) pour ce milieu.

Selon un autre aspect, l'invention concerne également les compositions comprenant une émulsion telle que définie précédemment. Ladite composition peut être à usage thérapeutique, cosmétique ou agro-alimentaire, et peut donc comprendre outre l'émulsion selon l'invention, tout autre composant entrant classiquement dans les compositions thérapeutiques, cosmétiques ou agro-alimentaires.

Selon un autre aspect, l'invention concerne également un procédé de préparation d'une émulsion telle que décrite précédemment. Ainsi, il est proposé un procédé de préparation d'une émulsion telle que définie précédemment caractérisé en ce qu'il comprend :
(i) ajouter un composé lipophile dans une solution aqueuse d'un polymère **I** à base d'unités cyclodextrines ou d'un polymère hydrophile **II** portant des cyclodextrines;
(ii) former une émulsion du mélange obtenu à l'étape (i).

Le polymère **I** à base d'unités cyclodextrines ou le polymère hydrophile **II** portant des cyclodextrines sont tels que définis précédemment.

Le composé lipophile est tel que défini précédemment.

En règle générale, le procédé de la présente invention est extrêmement simple de mise en oeuvre. Ainsi, l'étape (i) du procédé de l'invention peut consister en un simple mélange, qui est généralement conduit à température ambiante, c'est-à-dire le plus souvent entre 15°C et 30°C.

Quant à l'étape (ii) du procédé, celle-ci peut être mise en oeuvre par un moyen choisi dans le groupe comprenant la sonication (de préférence la sonication pulsée), l'homogénéisation par forces de cisaillement (par exemple avec un ultra-turrax®), et la microfluidisation (avec un microfluidisateur).

Le principe de fonctionnement d'un homogénéiseur est le suivant : par la haute vitesse du rotor, le fluide à travailler est aspiré automatiquement dans le sens axial dans la tête de dispersion et comprimé ensuite dans le sens radial à travers les fentes du système rotor/stator. Le matériel est ainsi soumis à de très hautes forces de cisaillement et de poussée. Dans l'interstice entre le rotor et le stator, il se forme en plus une haute turbulence donnant lieu à un mélange optimal de la suspension.

La microfluidisation permet de réduire physiquement des particules dispersées dans un liquide ou des gouttelettes à des tailles sub-micrométriques uniformes. La Microfluidisation est un nouveau type de process en biotechnologie, chimie, pharmacie, alimentaire. Typiquement, le produit à traiter pénètre dans le réservoir d'entrée; un système de pompe amplificatrice, pneumatique ou électro-hydraulique, générant une pression pratiquement continue (jusqu'à 2755 bar), accélère le flux à traiter jusqu'à des vitesses atteignant plusieurs centaines de mètres par seconde, le propulsant dans une zone de traitement appelée la chambre d'interaction. Grâce à la puissance du cisaillement et des impacts dans ces microcanaux, les particules ou les gouttelettes sont réduites à des tailles inférieures au micron.

Avantageusement, la formation de l'émulsion de l'étape (ii) peut être réalisée par sonication pulsée pendant une période de 10 secondes à 15 minutes, de préférence de 20 secondes à 5 minutes, de préférence de 30 à 50 secondes. La formation de l'émulsion de l'étape (ii) peut également être réalisée par ultra-turrax® pendant une période de 1 minute à 10 heures, de préférence de 5 minutes à 5 heures, de préférence de 10 minutes à 2 heures. La formation de l'émulsion de l'étape (ii) peut également être réalisée par passage d'une émulsion dite « grossière » (c'est-à-dire contenant de grosses gouttelettes obtenues par exemple par vortex) dans un microfluidisateur pendant une période de 10 secondes à 5 minutes, de préférence de 30 secondes à 3 minutes, de préférence de 45 secondes à 2 minutes, avantageusement de l'ordre de une minute.

A ce sujet, il faut bien souligner que le procédé de l'invention ne nécessite pas la mise en oeuvre de solvants ou de tensioactifs. De ce fait, une composition selon l'invention est, en général, exempte de toute trace de solvant organique et/ou d'agent tensioactif. Ainsi, selon un mode de réalisation, une émulsion selon l'invention peut comprendre de l'eau, des polymères I et/ou II et un composé lipophile tels que définis précédemment, à l'exclusion de tout autre composé.

Dans certains modes de réalisation, dans l'étape (i) du procédé, le composé lipophile est ajouté en tant que tel lorsqu'il se présente sous forme liquide. Alternativement, lorsque le composé lipophile se présente sous forme solide, celui-ci est ajouté en solution dans un solvant lipide non miscible à l'eau, tel que le miglyol®, le myristate d'isopropyle ou le squalène ; ou bien un corps gras liquide choisis dans le groupe comprenant les huiles naturelles d'origine végétale, animale ou marine (telles que l'huile d'olive, l'huile de sésame, l'huile d'argan, l'huile de palme, l'huile de soja, l'huile de pastel, l'huile de tortue, l'huile de babassu, l'aloe vera, l'huile d'avocat, l'allantoïne, le bisabol, l'huile de pépins de raisin, l'huile d'abricot, l'huile de germe de blé, l'huile d'amande, l'huile d'arachide, l'huile de noix de macadamia, l'huile d'argousier, l'huile d'onagre, l'huile de bourrache, l'huile de gingembre, le géraniol, l'huile de jujube, l'huile de vison, la lanoline), les huiles synthétiques, les huiles minérales (telles que l'isohexadécane, l'isoparaffin, la ceresin, la vaseline), les huiles hydrogénées, l'huile de silicone, les composés hydrocarbonés (tels que la paraffine liquide, les terpènes, le squalène), les acides gras saturés ou insaturés (tels que l'acide myristique), les esters d'acide gras, les cires liquides (comme l'huile de jojoba), les alcools gras (tels que l'alcool myristylique, l'alcool cétylique, l'alcool stéarylique, l'alcool myricylique), ou un mélange de ceux-ci.

Avantageusement, la concentration du polymère I à unités cyclodextrine ou du polymère hydrophile II portant des cyclodextrines dans la solution aqueuse de l'étape (i) est comprise entre 20 et 200 mg/ml, de préférence entre 25 et 175 mg/ml, de préférence entre 50 et 150 mg/ml, de préférence entre 90 et 110 mg/ml.

Avantageusement, dans l'étape (i), la quantité de composé lipophile est comprise entre 1 et 500 mg/ml, de préférence entre 1 et 400 mg/ml, de préférence entre 1 et 200 mg/ml, de préférence entre 1 et 100 mg/ml, de préférence entre 1 et 50 mg/ml, de préférence entre 1 et 25 mg/ml, de préférence entre 5 et 20 mg/ml de préférence entre 5 et 15 mg/ml.

Il est souvent avantageux que le ratio massique (Composé lipophile)/ (polymère I ou II) de la masse totale des composés lipophiles rapportée à la masse totale des polymères I ou II soit compris entre 1 % et 50 %, ce ratio étant de préférence supérieur à 2 %, et avantageusement supérieur à 3 %.

Le procédé selon l'invention peut comprendre en outre une étape d'augmentation de la teneur en composé lipophile de l'émulsion. Celle-ci peut être réalisée par ultracentrifugation de ladite émulsion. Cette étape peut être réalisée en centrifugeant une émulsion selon l'invention à au moins 40.000 rotations par minutes (rpm) pendant une période de temps allant de 5 minutes à 1 h et de préférence à 15 minutes. On observe alors deux phases : une première consistant en une solution aqueuse de polymères I ou polymère II, et une deuxième consistant en une émulsion concentrée qui se présente le plus souvent sous la forme d'une crème. Le diamètre hydrodynamique moyen des gouttelettes de l'émulsion ainsi obtenue est bien inférieur à celui des gouttelettes de l'émulsion avant ultracentrifugation. En moyenne, on passe d'un diamètre hydrodynamique moyen avant ultracentrifugation de l'ordre de 0,8 mm à 400 à 500 nm après ultracentifugation. La stabilité de l'émulsion ainsi ultracentrifugée s'en trouve augmentée.

Du fait de leur stabilité remarquable, les émulsions selon l'invention peuvent en général être soumises à une étape de lyophilisation.

Ainsi, le procédé selon l'invention peut comprendre en outre une étape de lyophilisation de l'émulsion. Celle-ci peut être réalisée par un lyophilisateur commercial. Le cas échéant, cette étape de lyophilisation peut généralement être conduite en refroidissant brutalement la composition (généralement dans l'air liquide ou l'azote liquide), puis en sublimant l'eau sous forte dépression. La lyophilisation peut être réalisée en utilisant les paramètres usuels de lyophilisation pour les mélanges ou solutions aqueux. Par exemple, la lyophilisation peut être mise en oeuvre sous un vide de 5 Pa à 30 Pa pendant une période comprise entre 24 et 80 heures, de préférence de l'ordre de 72 heures. A l'issue de la lyophilisation, une poudre qui se présente généralement sous la forme d'une poudre compacte d'aspect cotonneux est obtenue, laquelle peut être stockée pendant une durée indéterminée à température ambiante (soit entre 15 et 25°C) ou sous réfrigération. L'émulsion ainsi lyophilisée peut être ultérieurement reconstituée en dispersant le lyophilisat dans de l'eau. La quantité d'eau utilisée pour redisperser le lyohilisat peut être un volume équivalent à celui qui aura été retiré lors de l'étape de lyophilisation. En règle générale, la taille des gouttelettes de l'émulsion ainsi reconstituée est équivalente à celle de l'émulsion initiale. Alternativement, on pourra redisperser le lyophilisat dans un volume inférieur ou supérieur, selon que l'on souhaite une émulsion plus ou moins concentrée, respectivement.

Les compositions obtenues à l'issue d'une telle étape de lyophilisation, qui peuvent être dispersées dans l'eau pour conduire à la reconstitution d'une émulsion selon l'invention, constituent un autre objet particulier de la présente invention. Ainsi, selon un autre aspect, l'invention concerne une composition susceptible d'être obtenue par lyophilisation d'une émulsion telle que définie précédemment.

Selon encore un autre aspect, l'invention concerne une émulsion obtenue par un procédé tel que défini précédemment. L'invention concerne également l'utilisation d'une telle émulsion pour la fabrication d'une composition cosmétique, pharmaceutique ou agro-alimentaire.

Selon encore un autre aspect, l'invention concerne l'utilisation d'une émulsion obtenue par un procédé tel que défini précédemment pour réaliser une encapsulation concentrée de composés lipophiles. En effet, comme décrit précédemment, les inventeurs ont découvert qu'il était possible d'augmenter la teneur en composé lipophile dans l'émulsion selon l'invention par ultracentrifugation. Cet aspect trouve application dans le domaine cosmétique, en particulier dans le domaine des parfums. Le procédé selon l'invention permet en effet d'incorporer une quantité importante d'une haute concentration en composé lipophile odorant (e.g., parfum) dans une composition selon l'invention.

Egalement, ce dernier point s'applique à tous les autres principes actifs ou composés lipophiles susceptibles d'être encapsulés dans les cyclodextrines.

Selon encore un autre aspect, l'invention concerne l'utilisation d'une émulsion obtenue par un procédé tel que défini précédemment pour masquer l'odeur et/ou le goût d'un composé lipophile d'une composition cosmétique, pharmaceutique ou agro-alimentaire.

L'utilisation d'une émulsion susceptible d'être obtenue par un procédé tel que défini précédemment pour le relargage contrôlé d'un principe actif lipophile est également décrite.

L'utilisation d'une émulsion susceptible d'être obtenue par un procédé tel que défini précédemment pour prolonger l'effet cosmétique ou thérapeutique d'un composé d'une composition cosmétique ou pharmaceutique **est également décrite.** Il peut s'agir par exemple de l'effet d'un déodorant, de l'effet de répulsion d'un anti-moustique,...

Selon encore un autre aspect, l'invention concerne l'utilisation d'une émulsion obtenue par un procédé tel que défini précédemment pour :
- atténuer ou masquer l'odeur d'un composé lipophile composant ladite émulsion,
- atténuer ou masquer le mauvais goût d'un composé lipophile composant ladite émulsion,
- améliorer la stabilité des composés lipophiles facilement oxydables,
- améliorer la stabilité des composés lipophiles facilement volatiles, et/ou
- augmenter la solubilité des composés lipophiles insolubles.

Selon encore un autre aspect, l'invention concerne l'utilisation d'un complexe d'inclusion non-covalent et non-cristallin formé (i) d'un polymère à base d'unités cyclodextrine ou d'un polymère hydrophile portant des cyclodextrines et (ii) d'un composé lipophile, pour stabiliser une émulsion, le polymère à base d'unités cyclodextrine, le polymère hydrophile portant des cyclodextrines et le composé lipophile étant tels que définis précédemment ; **dans lequel complexe d'inclusion, le polymère à base d'unités cyclodextrine et le polymère hydrophile portant des cyclodextrines contiennent au moins 10 unités cyclodextrine.**

Ainsi, selon le procédé de l'invention, il est possible de préparer de façon très simple et avec un faible apport énergétique des émulsions huile-dans-l'eau, eau-dans-l'huile, simples ou multiples, extrêmement stables.

La stabilité des émulsions selon l'invention peut notamment être mise en évidence par la conservation du diamètre hydrodynamique moyen des gouttelettes de la phase dispersée suite à un stockage. Ainsi, en général, suite à un stockage de 24 heures, le diamètre hydrodynamique moyen des gouttelettes d'une émulsion selon l'invention reste inférieur ou égal à 5000 nm. Le plus souvent, suite à un stockage de 24 heures, le diamètre hydrodynamique moyen des gouttelettes d'une émulsion selon l'invention reste inférieur ou égal à 800 nm, de préférence inférieur ou égal à 500 nm, avantageusement inférieur ou égal à 400 nm, et encore plus avantageusement inférieur ou égal à 300 nm. De préférence, le diamètre hydrodynamique moyen des gouttelettes d'une émulsion selon l'invention reste inférieur ou égal à 500 nm, et de préférence inférieur ou égal à 400 nm, suite à un stockage de 2 jours, voire suite à un stockage de 5 jours, voire suite à un stockage de 15 jours, voire suite à un stockage de 25 jours, et même, dans certains cas, après un stockage de 60 jours. Il est toutefois à souligner que, pour observer une bonne conservation du diamètre hydrodynamique moyen des gouttelettes suite à un stockage prolongé, il peut parfois être nécessaire de conserver l'émulsion en atmosphère non oxydante (sous argon par exemple) de façon à éviter un vieillissement de ses constituants, qui est susceptible de remettre en cause la stabilité du système.

Le terme de "diamètre hydrodynamique moyen", au sens où il est employé dans la présente description, désigne la taille d'une gouttelette d'huile au sein d'un milieu aqueux (émulsions huile-dans-l'eau) ou d'une gouttelette d'eau au sein d'un milieu huileux (émulsions eau-dans-l'huile), qui prend en compte le diamètre moyen de la gouttelette dans sa conformation en milieu aqueux ou huileux, ainsi que son éventuelle couche de solvatation. Le diamètre hydrodynamique moyen d'une population de gouttelettes au sein d'un milieu aqueux ou huileux peut notamment être déterminé par diffusion quasi élastique de la lumière au sein du milieu considéré, notamment par un appareil de type Nanosizer. Ce type d'appareil permet également de déterminer pour la population de gouttelettes un indice de polydispersité du diamètre hydrodynamique, qui reflète la distribution des diamètres hydrodynamiques, plus ou moins resserrée autour de la valeur moyenne.

Dans certains modes de réalisation, l'émulsion selon la présente demande ne contient pas de composés cyclodextrine autres que le polymère **I** à base d'unités cyclodextrine ou le polymère hydrophile **II** portant des cyclodextrines tels que définis dans le présent document, ceci pouvant s'appliquer indépendamment à chacun des modes de réalisation par ailleurs décrits dans le présent document. Par exemple, l'émulsion selon la présente demande ne contient pas de monomères cyclodextrine, par exemple des cyclodextrines hydroxyalkylées telles que celles décrites dans le document JP 03/058906. Par exemple, l'émulsion selon la présente demande ne contient pas non plus d'oligomères de cyclodextrine, c'est-à-dire des composés contenant 2 à 6 voire 3 à 4 cyclodextrines réticulées ou connectées entre elles, éventuellement par un groupe espaceur (« linker »), telles que les trimères ou tétramères de beta-cyclodextrine décrits dans le document JP 61/227517.

Ainsi, selon certains modes de réalisation, une émulsion selon l'invention peut comprendre de l'eau, des polymères **I** et/ou **II** et un composé lipophile tels que définis précédemment, à l'exclusion de monomères ou oligomères de cyclodextrine tels que définis dans le paragraphe précédent.

D'autres caractéristiques, aspects et avantages de la présente invention apparaitront à la lecture des exemples illustratifs exposés ci-après et des figures annexées.

### Brève description des figures

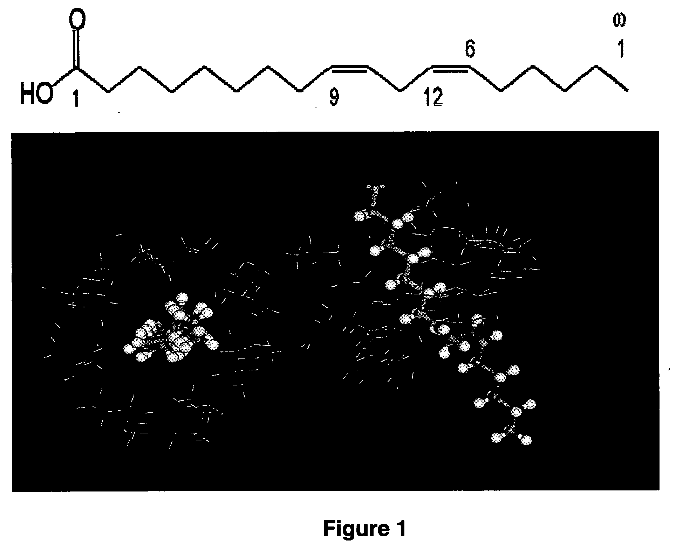

- La figure 1 représente une modélisation moléculaire du système Parsol® MCX/ dimère de β-cyclodextrines.

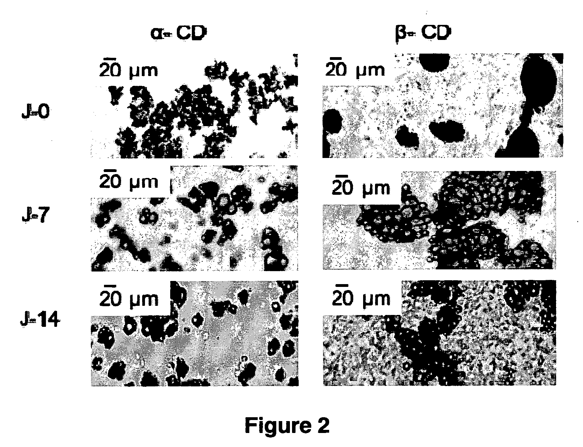

- La figure 2 représente des clichés de microscopie optique d'un précipité insoluble obtenu après émulsion d'une solution aqueuse de α-cyclodextrine (α-CD) et de Parsol® MCX ou d'une solution aqueuse de β-cyclodextrine (β-CD) et de Parsol® MCX le jour de préparation des émulsions (J-0), sept et quatorze jours après (J-7 et J-14 respectivement).

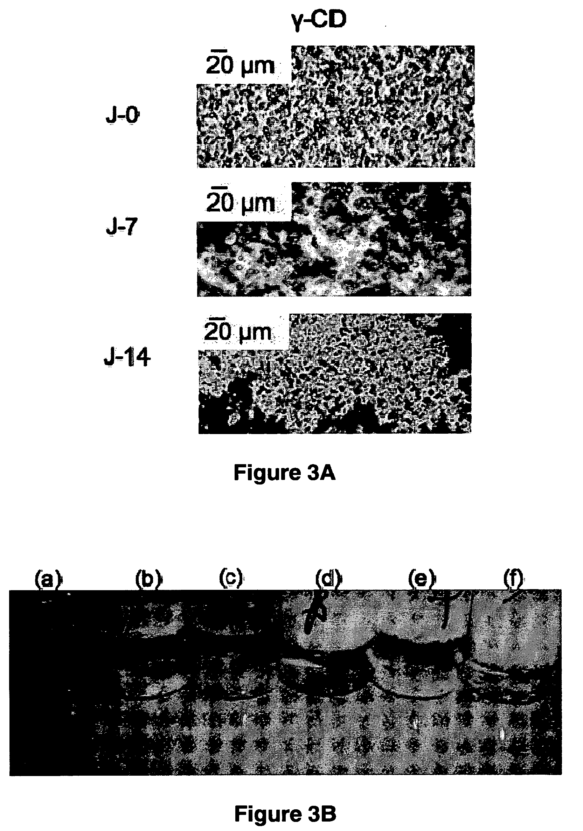

- La figure 3A représente des clichés de microscopie optique d'une émulsion huile-dans-l'eau obtenue à partir d'une solution aqueuse de γ-cyclodextrines (γ-CD) et de Parsol® MCX le jour de préparation de l'émulsion (J-0), ainsi qu'un précipité insoluble obtenu sept et quatorze jours après la préparation de l'émulsion précitée (J-7 et J-14 respectivement).
- La figure 3B représente une photographie des échantillons présentant un précipité insoluble obtenu après la préparation d'une émulsion à partir de : (a) solution aqueuse de α-cyclodextrines et Géraniol, (b) solution aqueuse de α-cyclodextrines et un concentré de parfum, (c) solution aqueuse de α-cyclodextrines et de l'huile de Gingembre, (d) solution aqueuse de β-cyclodextrines et du Géraniol, (e) solution aqueuse de β-cyclodextrines et un concentré de parfum ou (f) solution aqueuse de β-cyclodextrines et de l'huile de Gingembre.

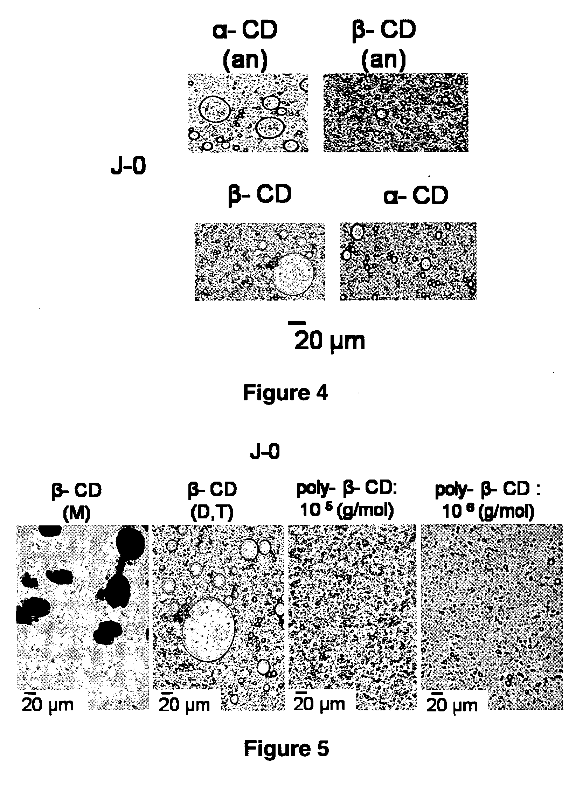

- La figure 4 représente des clichés de microscopie optique d'émulsions huile-dans-l'eau obtenues à partir d'une solution aqueuse de dimères/trimères de α, β, γ-cyclodextrines (α, β, γ-CD) et de Parsol® MCX le jour de préparation des émulsions (J-0). « an » signifie anionique
- La figure 5 représente des clichés de microscopie optique des précipités et des émulsions huile-dans-l'eau obtenus à partir d'une solution aqueuse de monomères (M) dimères (D), trimères (T) ou polymères (poly) de β-cyclodextrine (β-CD) et du Parsol® MCX le jour de préparation des émulsions (J-0).

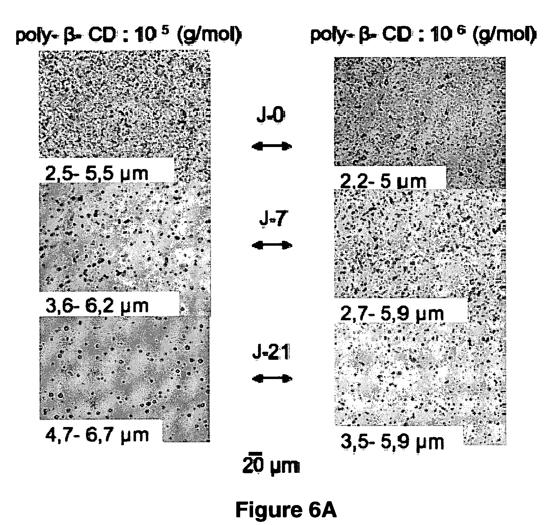

- La figure 6A représente des clichés de microscopie optique d'émulsions huile-dans-l'eau obtenues à partir d'une solution aqueuse de polymères de β-cyclodextrine (poly-β-CD) de masse molaire moyenne 10⁵ et 10⁶ g/mol et de Parsol® MCX le jour de préparation des émulsions (J-0), sept jours après préparation (J-7) et vingt-et-un jours après la préparation (J-21). Les diamètres hydrodynamiques moyens des gouttelettes sont indiqués sur les clichés.

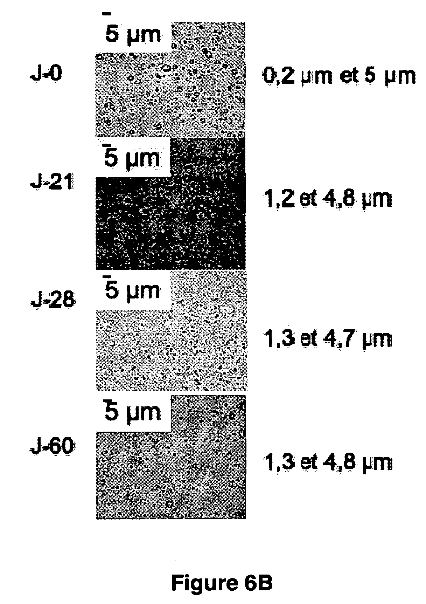

- La figure 6B représente des clichés de microscopie optique d'émulsions huile-dans-l'eau obtenues à partir d'une solution aqueuse de polymères de β-cyclodextrine (poly-β-CD) à 100 mg/mL et de l'huile de Bourrache le jour de préparation des émulsions (J-0) et vingt-et-un, vingt-huit et soixante jours après préparation (J-21, J-28, J-60 respectivement). Les diamètres hydrodynamiques moyens des gouttelettes sont indiqués en vis-à-vis des clichés. Les émulsions sont obtenues par solubilisation d'un polymère de β-cyclodextrines de masse molaire moyenne de 10⁵ g/mol dans de l'eau en une concentration de 100 mg/mL. 20 mg d'huile de bourrache sont ajoutés à 2 mL de la solution aqueuse de poly-β-cyclodextrine ainsi préparée. Le mélange est ensuite soumis à sonication pulsée pendant 40 secondes. L'émulsion ainsi obtenue est conservée à 25°C sous agitation tangentielle. Ce résultat illustre la stabilité remarquable de l'émulsion d'huile de bourrache au cours du temps (60 jours).

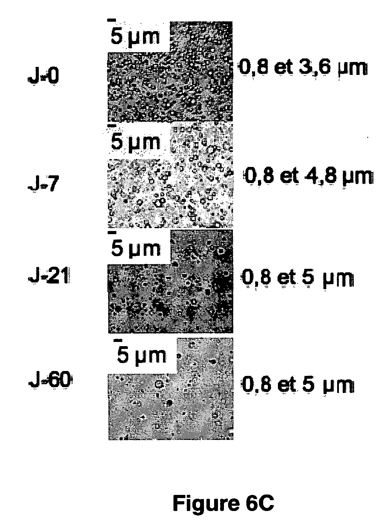

- La figure 6C représente des clichés de microscopie optique d'émulsions huile-dans-l'eau obtenues à partir d'une solution aqueuse de polymères de β-cyclodextrine (poly-β-CD) à 100 mg/mL et de l'huile de Gingembre le jour de préparation des émulsions (J-0) et sept, vingt-et-un et soixante jours après préparation (J-7, J-21, J-60 respectivement). Les diamètres hydrodynamiques moyens des gouttelettes sont indiqués en vis-à-vis des clichés. Les émulsions sont obtenues par solubilisation d'un polymère de β-cyclodextrines de masse molaire moyenne de 10⁵ g/mol dans de l'eau en une concentration de 100 mg/mL. 20 mg d'huile de Gingembre sont ajoutés à 2 mL de la solution aqueuse de poly-β-cyclodextrine ainsi préparée. Le mélange est ensuite soumis à sonication pulsée pendant 40 secondes. L'émulsion ainsi obtenue est conservée à 25 °C sous agitation tangentielle. Ce résultat illustre la stabilité remarquable de l'émulsion d'huile de Gingembre au cours du temps (60 jours).

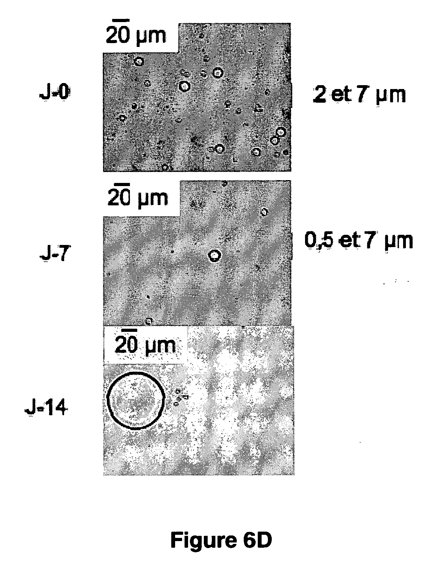

- La figure 6D représente des clichés de microscopie optique d'émulsions huile-dans-l'eau obtenues à partir d'une solution aqueuse de polymères de γ-cyclodextrine (poly-γ-CD) de masse molaire moyenne 2.10⁶ g/mol à une concentration de 100 mg/mL et de l'huile de Gingembre le jour de préparation des émulsions (J-0) et sept et quatorze jours après préparation (J-7, J-14 respectivement). Les diamètres hydrodynamiques moyens des gouttelettes sont indiqués en vis-à-vis des clichés. Les émulsions sont obtenues par solubilisation d'un polymère de γ-cyclodextrines de masse molaire moyenne de 2.10⁶ g/mol dans de l'eau en une concentration de 100 mg/mL. 20 mg d'huile de Gingembre sont ajoutés à 2 mL de la solution aqueuse de poly-γ-cyclodextrine ainsi préparée. Le mélange est ensuite soumis à sonication pulsée pendant 40 secondes. L'émulsion ainsi obtenue est conservée à 25°C sous agitation tangentielle. Ce résultat illustre l'instabilité de l'émulsion obtenue avec le polymère de γ-cyclodextrine du fait du diamètre de cavité trop important (le complexe d'inclusion formé avec le principe actif lipophile (huile de gingembre) n'est pas stable).

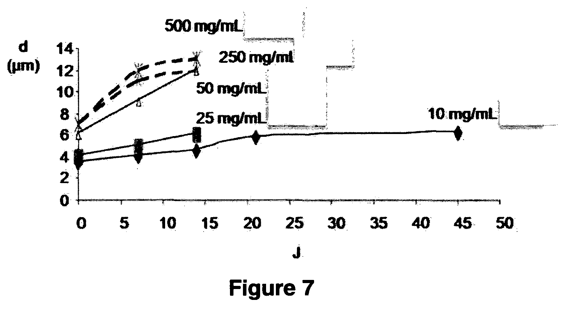

- La figure 7 représente la variation dans le temps de la taille des gouttelettes d'émulsions préparées à partir d'une solution aqueuse de 100 mg/mL de poly-β-cyclodextrine et de Parsol® MCX, en fonction de la concentration en Parsol® MCX. En abscisse, « J » représente le nombre de jours. En ordonnée, « d » représente le diamètre hydrodynamique moyen des gouttelettes en µm. La concentration en Parsol® MCX est exprimée en mg/mL.

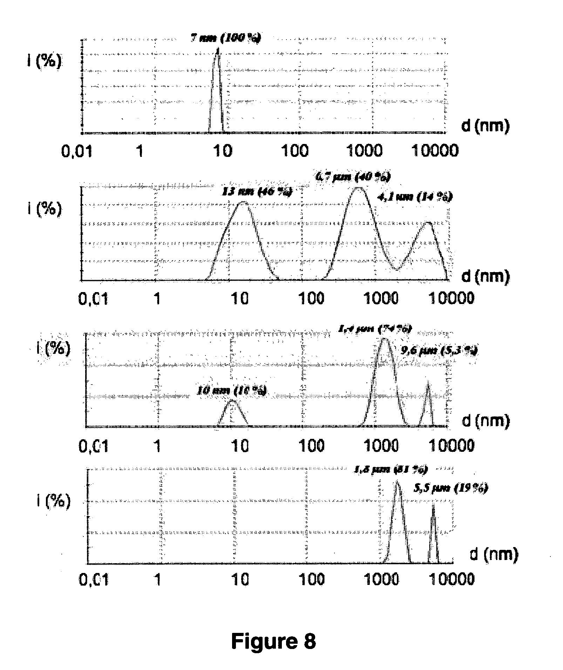

- La figure 8 représente les résultats obtenus par diffraction dynamique de la lumière (DLS) sur des systèmes de poly-β-cyclodextrines, et des gouttelettes de Parsol® MCX stabilisées avec les poly-β-cyclodextrines avec en :
   ∘ (a) : poly-β-cyclodextrines pure à 100 mg/mL ;
   ∘ (b) : poly-β-cyclodextrines à 100 mg/mL et Parsol® MCX à 2 mg/mL;
   ∘ (c) : poly-β-cyclodextrines à 100 mg/mL et Parsol® MCX à 5 mg/mL;
   ∘ (d): poly-β-cyclodextrines à 100 mg/mL et Parsol® MCX à 10 mg/mL;
   Où, en abscisse, « d » représente le diamètre en nanomètre (nm), et en ordonnée, « i » représente l'intensité en pourcentage.
- La figure 9 représente une comparaison entre des clichés de microscopie optique d'émulsions huile-dans-l'eau obtenues à partir d'une solution aqueuse de 100 mg/mL de polymères β-cyclodextrine (poly-β-CD) à une masse molaire moyenne de 10⁵ et de 10⁶ g/mol et de 10 µg/mL de Parsol® MCX, le jour (J-0) et sept jours (J-7) après la préparation des émulsions.
- La figure 10 représente une comparaison entre les clichés de microscopie optique des émulsions huile-dans-l'eau obtenues à partir d'une solution aqueuse de 100 mg/mL de polymères β-cyclodextrine (poly-β-CD) et de 10µL/mL de concentré de parfum ou d'huile essentielle de Gingembre, le jour (J-0) et sept jours (J-7) après la préparation des émulsions.
- La figure 11 représente une comparaison entre les clichés de microscopie optique des émulsions huile-dans-l'eau obtenues à partir d'une solution aqueuse de 100 mg/mL de polymères β-cyclodextrine (poly-β-CD) et de 10µL/mL de d'huile essentielle de Lavandin ou d'huile essentielle de Bourrache, le jour (J-0) et sept jours (J-7) après la préparation des émulsions.
- La figure 12 des clichés de microscopie optique des émulsions huile-dans-l'eau obtenues à partir d'une solution aqueuse de 100 mg/mL des polymères β-cyclodextrine (poly-β-CD) et de 10µL/mL de Géraniol, le jour (J-0) et sept jours (J-7) après la préparation des émulsions.
- La figure 13 représente un schéma de concentration des émulsions par ultracentrifugation (étape (a)) et redispersion (étape (b)). « Cr » signifie crème très dense.
- La figure 14 représente des clichés de microscopie optique d'une crème de Parsol® MCX obtenue après ultracentrifugation (a) et redispersion (b) d'une émulsion huile-dans-l'eau obtenue à partir de 2 mL d'une solution aqueuse de 100 mg/mL de poly-β-cyclodextrine et 10 mg/mL de Parsol® MCX.

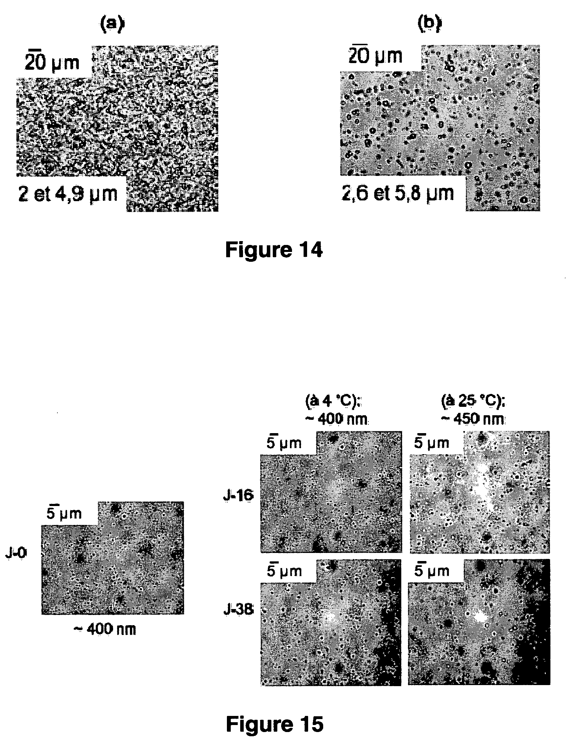

- La figure 15 représente des clichés de microscopie optique d'une émulsion concentrée à base de polymère de β-cyclodextrine (poly-β-CD) et de Géraniol (émulsion huile-dans-l'eau poly-β-CD/Géraniol avec une teneur accrue en huile de Géraniol), le jour de préparation des émulsions concentrées (J-0) et seize et trente-huit jours (J-16 et J-38 respectivement) après la préparation des émulsions concentrées, à 4°C et à 25°C. Les émulsions concentrées de Géraniol sont obtenues par solubilisation d'un polymère de β-cyclodextrines de masse molaire moyenne de 10⁵ g/mol dans de l'eau en une concentration de 100 mg/mL. 50 mg d'huile de Géraniol sont ajoutés à 5 mL de la solution aqueuse de poly-β-cyclodextrine ainsi préparée. Le mélange est ensuite soumis à sonication pulsée pendant 40 secondes. L'émulsion ainsi obtenue est soumise à ultracentrifugation à 40 000 tours/seconde pendant 1 heure. Le concentré de Géraniol ainsi obtenu est séparé avec une pipette Pasteur (1 mL). Nous observons la formation de gouttelettes de diamètre < 500 nm. Cependant l'émulsion ainsi formée (le concentré de Géraniol) est stable dans le temps.

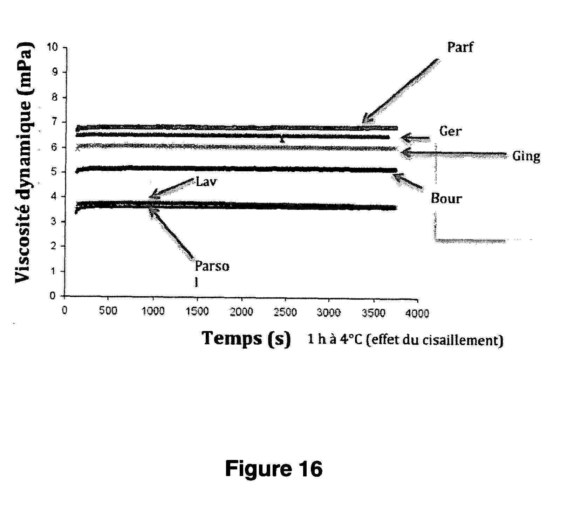

- La figure 16 représente les résultats obtenus lors d'une étude rhéologique (évolution de la viscosité dynamique (mPa) en fonction du temps (secondes)) réalisé sur des émulsions huile-dans-l'eau obtenues à partir de 2 mL d'une solution aqueuse de polymère de β-cyclodextrine (poly-β-CD) (100 mg/mL) et 10 mg/mL d'huile de Bourrache (Bour), d'huile de Gingembre (Ging), de concentré de parfum (Parf), d'huile de Lavandin (Lav), de Géraniol (Ger) ou de Parsol® MCX (Parsol) à 4°C. En abscisse « T » représente le temps en secondes (s). En ordonnée, « Vd » représente la viscosité dynamique en millipascal (mPa). Les émulsions sont obtenues par solubilisation d'un polymère de β-cyclodextrines de masse molaire moyenne de 10⁵ g/mol dans de l'eau en une concentration de 100 mg/mL. 20 mg de principe actif lipophile (huile de Géraniol, Gingembre, Bourrache, Lavandin, concentré de parfum ou Parsol® MCX) sont ajoutés à 2 mL de la solution aqueuse de poly-β-cyclodextrine ainsi préparée. Le mélange est ensuite soumis à sonication pulsée pendant 40 secondes. Les émulsions ainsi préparées ont été étudiées avec un appareil rhéologique RS600 muni d'un Géomètre cône-plan, diamètre 60 mm, angle 1°,7200 s-1; à 4 °C pendant une période de 3600 secondes. Les résultats obtenus indiquent la stabilité remarquable de ces émulsions (la viscosité dynamique reste constante) soumis à un effet de cisaillement pendant une période très longue (1 heure).
- La figure 17 des clichés de microscopie optique d'une émulsion eau-dans-l'huile préparée à partir de 2 mL d'une solution aqueuse de polymère β-cyclodextrine (poly-β-CD) à une masse molaire moyenne de 10⁵ g/mol (10 mg de poly-β-CD dissout dans 100 mL d'eau) et de 2 mL de Parsol® MCX.
- La figure 18 représente des images en microscopie optique des émulsions à base de polymère de β-cyclodextrine (poly-β-CD) préparées selon l'Exemple 7: (a) Squalène ; (b) huile de Gingembre, (c) huile de Bourrache.
- Les figures 19 A et 19 B représentent une comparaison rhéologique interfaciale d'une goutte d'une émulsion préparée à partir de Squalène et (a) d'une solution aqueuse de monomères de β-cyclodextrine (β-CD) (Figure 19A) ou (b) d'une solution aqueuse de polymère de β-cyclodextrine (poly-β-CD) (Figure 19B).
- La figure 19 C représente une variation sinusoïdale de la tension interfaciale avec le volume de la goutte de Squalène dans le système Squalène/ polymère de β-cyclodextrine (poly-β-CD) de la Figure 19 B (rhéologie interfaciale).
- La figure 20 représente des images en microscopie optique des émulsions à base de sébum synthétique et de polymère de ß-cyclodextrine (poly-ß-CD) préparées selon l'Exemple 8.
- La figure 21 représente des images en microscopie optique des émulsions à base des mélanges Squalène/huile de géranium (50/50) (w/w) et polymère de α-cyclodextrine (poly-α-CD) obtenues selon l'Exemple 9.

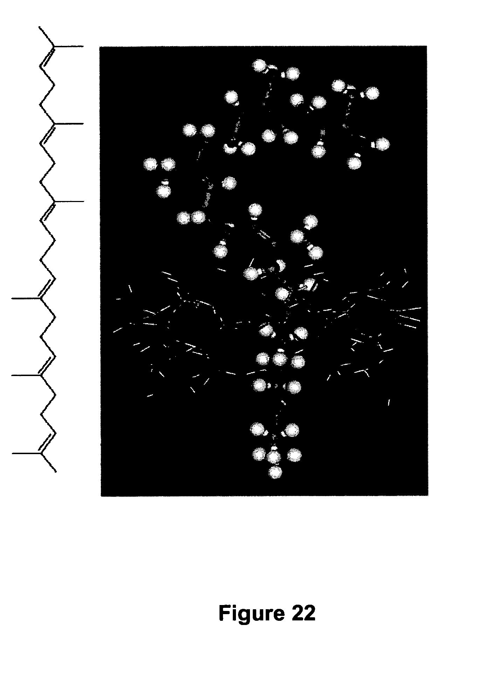

- La figure 22 représente une modélisation moléculaire du complexe hôte-invité amphiphile Squalène/ polymère de ß-cyclodextrine (poly-ß-CD).

### EXEMPLES

### Exemple comparatif 1 - « Emulsions » à partir de monomères de cyclodextrines

Par rapport aux travaux déjà relatés dans la littérature [1-8], on observe que l'addition des monomères de cyclodextrines dans les émulsions doubles (multiples) entraine une stabilisation importante du système. À partir de ces observations, plusieurs essais avec ces trois monomères (α-, β- et γ-cyclodextrines) ont été réalisés afin d'augmenter la stabilité des émulsions à base d'une huile de Parsol® MCX (Filtre anti-UV).

Dans un premier temps, une étude par modélisation moléculaire préliminaire à la partie expérimentale a été réalisée. Il a été ainsi observé (Figure 1) avec un dimère de cyclodextrine qu'une interaction était possible avec deux molécules de Parsol® MCX.

Des tentatives de préparation d'émulsions ont été réalisées en trois étapes comme suit :
(1) solubilisation de la β cyclodextrine (β-CD) (17 mg/mL) dans l'eau (facteur limitatif : la solubilité de la β-CD);
(2) rajout de l'huile (Parsol® MCX), (10 µL pour 1 mL de solution aqueuse de β cyclodextrine)
(3) sonication en bain glacé (40 sec, pulsée).

Brièvement, le protocole suivant a été utilisé :
Les monomères de α-, β- ou γ-cyclodextrines sont mis en solution dans de l'eau en une concentration de 17 mg/mL. 10 mg de Parsol® MCX sont ajoutés à 1 mL de la solution aqueuse de α-, β- ou γ-cyclodextrine ainsi préparée. Le mélange est ensuite soumis à sonication pulsée pendant 40 secondes en un bain glacé (0°C). Le produit ainsi obtenu est conservé à 25°C sous agitation tangentielle.

Cependant, les tentatives de préparation d'émulsions à partir des monomères (α-, β-) de cyclodextrines conduisent à la formation d'un précipité insoluble (Figure 2). Les γ- cyclodextrines conduisent à la formation d'une émulsion dans un premier temps (pendant deux jours) puis une précipitation (Figure 3A).

D'autres produits ont été testés de la même manière (avec les monomères α- et β), tels que le Géraniol, l'huile de Gingembre ou un concentré de parfum. Ainsi, des tentatives de préparation d'émulsions ont été réalisées à partir des composants suivants, en utilisant le même protocole que celui décrit pour le Parsol® MCX ci-dessus :
(a) solution aqueuse de α-cyclodextrines et Géraniol,
(b) solution aqueuse de α-cyclodextrines et un concentré de parfum,
(c) solution aqueuse de α-cyclodextrines et de l'huile de Gingembre,
(d) solution aqueuse de ß-cyclodextrines et du Géraniol,
(e) solution aqueuse de ß-cyclodextrines et un concentré de parfum ou
(f) solution aqueuse de ß-cyclodextrines et de l'huile de Gingembre.

Cependant dans tous les cas, la formation d'un précipité insoluble a été observée très rapidement (à partir du premier jour) (Figure 3B).

Les monomères de cyclodextrines ne permettent donc pas de former des émulsions. On obtient des précipités et non des émulsions.

### Exemple comparatif 2 - « Emulsions » à partir d'oligomères de cyclodextrines

À la suite de ce résultat peu encourageant (formation d'un précipité) avec l'emploi de cyclodextrines, des essais ont été menés avec la série suivante de dimères/trimères (D/T) (% massique de cyclodextrines dans le polymère déterminé par RMN = 50 % en poids par rapport au poids total de oligomère) de provenance CycloLab Cyclodextrin Research & Development Laboratory, Ltd. Budapest, Hungary:
- (D, T) α -cyclodextrine anionique,
- (D, T) α -cyclodextrine sulfaté,
- (D, T) γ -cyclodextrine,
- (D, T) β cyclodextrine anionique,
- (D, T) β -cyclodextrine,
- (D, T) γ -cyclodextrine anionique,
- (D, T) β -cyclodextrine sulfaté,
- (D, T) α -cyclodextrine.

Le protocole suivant a été utilisé pour préparer les différentes émulsions/précipités :
Les dimères /trimères de α-, β- ou γ-cyclodextrines sont mis en solution dans de l'eau. 10 mg de Parsol® MCX sont ajoutés à 1 mL de la solution aqueuse dimère /trimère de α-, β- ou γ-cyclodextrine ainsi préparée. Le mélange est ensuite soumis à sonication pulsée pendant 40 secondes en un bain glacé (0°C). Le produit ainsi obtenu est conservé à 25°C sous agitation tangentielle.

L'observation par microscopie optique indique dans tous les cas la formation d'une émulsion avec une taille inhomogène, de gouttelette très variable (très larges) mais une coalescence rapide après trois-quatre jours.

Les oligomères de cyclodextrines ne permettent donc pas de former des émulsions stables.

Au vu de ces résultats, il semble important de souligner l'influence de la masse moléculaire et de la taille de la cavité des unités cyclodextrine, dans la stabilité des émulsions. Comme montré dans l'exemple comparatif 1 ci-dessus, concernant les monomères -α (masse moléculaire = 972 g/mol avec un diamètre de la cavité intérieure de 4,7- 5,2 (Å)) et -β (masse moléculaire = 1135 g/mol avec un diamètre de la cavité intérieure de 6,0- 6,4 (Å)), un précipité insoluble est formé dès le premier jour (jour zéro). Cependant, le monomère -γ (masse moléculaire = 1297 g/mol avec un diamètre de la cavité intérieure de 7,5- 8,3 (Å)) forme une émulsion dans un premier temps puis précipite rapidement (deux jours). Les essais réalisés avec les dimères/trimères à base de α, β et γ- cyclodextrines dans l'exemple comparatif 2 indiquent la formation des émulsions sans précipitation, mais avec une coalescence rapide. Ainsi, la masse moléculaire et la taille de la cavité intérieure, des différentes cyclodextrines employées (monomères, dimères/trimères) semblent avoir un effet sur la stabilité des émulsions, sans toutefois conduire à une stabilité viable pour des applications cosmétiques, pharmaceutiques et/ou agro-alimentaires.

### Exemples portant sur des émulsions à base de polycyclodextrines

Des essais ont été réalisés avec des polymères de cyclodextrine de masse moléculaire importante (10⁵ et 10⁶ g/mol).

### Exemple 1 : synthèse de polymères à base d'unités de β-cyclodextrine

Des polymères à base d'unités cyclodextrines ont été préparés en faisant réagir de la β-cyclodextrine (notée β-CD) avec de l'épichlorhydrine en milieu basique, selon le mode opératoire décrit dans l'European Polymer Journal, vol 33, No 1, pp 49-57 (1997). La masse molaire du polymère peut être modulée en variant la quantité d'épichlorhydrine utilisée pour la polycondensation.

### 1. 1-Synthèse d'un poly-β-cyclodextrine de masse molaire 40 000 g/mol

Dans un bicol, on a dissous 5g de β-CD dans une solution aqueuse de soude à 33% en masse. On a laissé ce mélange sous agitation, à température ambiante (20°C), pendant 24 h, de façon à réaliser la déprotonation des groupes hydroxyles.

On a ensuite introduit 2,7 mL d'épichlorhydrine dans le milieu (rapport molaire β-CD/épichlorhydrine = 1/7), et le mélange a été agité vigoureusement et porté à 30°C. On a laissé le milieu dans ces conditions pendant 3 heures.

La réaction a alors été arrêtée, par addition d'acétone qui dissout l'excès d'épichlorhydrine. La solution d'acétone surnageante a ensuite été éliminée.

Le polymère (précipité) est dissous dans de l'eau distillée et la solution a été portée à pH 12, et a été placée sous agitation pendant 24 h. La valeur du pH a ensuite été ramenée à 7 (par ajout d'acide chlorhydrique 6N), puis le mélange a été ultrafiltré avec une membrane de seuil de coupure de 1000 Dalton, afin d'éliminer les sels.

Le polymère obtenu suite à ces différentes étapes a ensuite été lyophilisé puis conservé au congélateur. La masse moléculaire de 40 000 g/mol a été déterminée par chromatographie SEC, avec un étalonnage pullulan. Le nombre moyen d'unités β-CD par polymère calculé à partir de cette masse moléculaire est de 20.

### 1. 2-Synthèse d'un poly-β-cyclodextrine de masse molaire 2 600 000 g/mol

Comme pour le polymère préparé au point 1.2 ci-dessus, on a dissous dans un bicol 5g de β-CD dans une solution aqueuse de soude à 33% en masse. On a laissé ce mélange sous agitation, à température ambiante (20°C), pendant 24 h, de façon à réaliser la déprotonation des groupes hydroxyles.

On a ensuite introduit 3,8 mL d'épichlorhydrine dans le milieu (rapport molaire β-CD/épichlorhydrine = 1/10), et le mélange a été agité vigoureusement et porté à 30°C. On a laissé le milieu évoluer jusqu'au voisinage immédiat de la gélification du milieu réactionnel, c'est-à-dire jusqu'à obtention d'un milieu de haute viscosité.

La réaction a alors été arrêtée, par addition d'acétone qui dissout l'excès d'épichlorhydrine. La solution d'acétone surnageante a ensuite été éliminée.

Le polymère (précipité) est dissous dans de l'eau distillée et la solution a été portée à pH 12, et a été placée sous agitation pendant 24 h. La valeur du pH a ensuite été ramenée à 7 (par ajout d'acide chlorhydrique 6N), puis le mélange a été ultrafiltré avec une membrane de seuil de coupure de 1000 Dalton, afin d'éliminer les sels. Une deuxième ultrafiltration a ensuite été réalisée avec une membrane de seuil de coupure 100 000 Dalton de façon à éliminer les fractions de faible masse molaire.

Le polymère obtenu suite à ces différentes étapes a ensuite été lyophilisé puis conservé au congélateur. La masse moléculaire de 2 600 000 g/mol a été déterminée par chromatographie SEC, avec un étalonnage pullulan. Le nombre moyen d'unités β-CD par polymère calculé à partir de cette masse moléculaire est de 1350.

Il est entendu que les poly-α- et poly-γ-cyclodextrines peuvent être obtenues par des protocoles similaires en substituant la β-CD par la α-CD ou γ-CD.

Dans le présent document, le sigle « CD » se réfère à la cyclodextrine. Ainsi, α-CD, β-CD et γ-CD signifient monomères de α-cyclodextrine, β-cyclodextrine et γ-cyclodextrine, respectivement. De la même manière, poly-α-CD, poly-β-CD et poly-γ-CD signifient polymères de α-cyclodextrine, β- cyclodextrine et γ-cyclodextrine, respectivement.

### EXEMPLE 2 : préparation d'émulsions (formulations) antisolaires (Parsol® MCX) par voie directe (huile-dans-l'eau) selon le procédé de la présente invention.

Des émulsions par voie directe correspondant à une quantité totale égale à 2 mL ont été préparées en utilisant des polymères à base de β-cyclodextrines avec des différentes valeurs de masses molaires (10⁵ et 10⁶ g/mol).

Les polymères de cyclodextrines sont solubilisés dans l'eau à raison de 100 mg/mL (le protocole peut être adapté en variant la concentration en polycyclodextrine entre 20 mg/mL et 200 mg/mL).

Une quantité variable de l'agent anti-UV (Parsol® MCX) est ajoutée dans 2 mL de la solution de polymères de cyclodextrines à raison de 10 mg de Parsol® MCX par mL de solution de polycyclodextrine (le protocole peut être adapté en variant la concentration en Parsol® MCX entre 2,5 mg et 500 mg par mL de solution de polycyclodextrine).

L'émulsion est formée par sonication pulsée pendant une période de 40 secondes ou par l'utra-turrax® pendant une période variable allant de quelques minutes jusqu'à deux heures.

Les émulsions ainsi obtenues sont conditionnées pour la conservation à 25°C, par agitation tangentielle, à 37 °C ou 4°C pendant une période variable allant de quelques jours jusqu'à quelques mois avant utilisation.

De manière générale on obtient ainsi une émulsion fluide homogène qui est de couleur blanche et qui est stable dans un rapport molaire β-CD/Parsol® MCX de 1/ 2,5, 1/1 et 1/ 0,5.

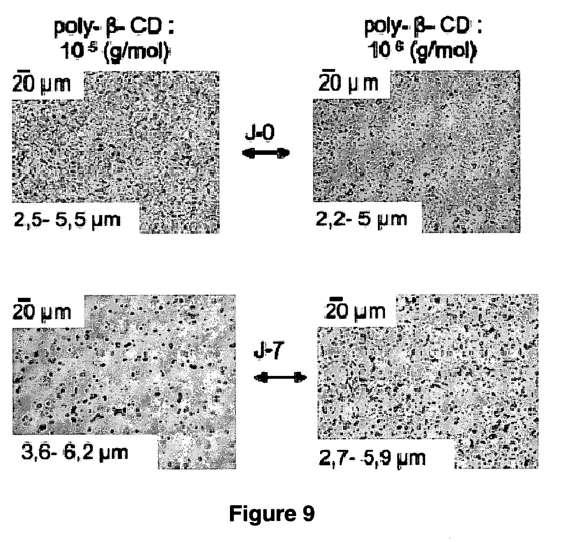

L'examen au microscope optique (observation de la morphologie et analyse d'image) ainsi par les techniques de diffusion dynamique de la lumière (DLS, granulométrie laser) montrent que les émulsions ainsi préparées forment des gouttelettes sphériques, très bien définies, avec une dimension < 10 µm (Figure 9).

La répartition granulométrique de l'émulsion ainsi obtenue est stable dans le temps ne présentant pas de phénomène de coalescence.

Un paramètre étudié a été la concentration en polymère. Plusieurs essais ont été effectués à des concentrations variables afin d'obtenir des émulsions avec une taille de gouttelette stable dans le temps. Globalement, l'augmentation de la concentration de polymère de β-cyclodextrine dans le système à partir de 20 mg/mL jusqu'à 200 mg/mL et de préférence 100 mg/mL permettent l'obtention d'une émulsion stable dans le temps.

Des études rhéologiques sur deux systèmes contenant 50 et 100 mg/mL poly-β-cyclodextrines et 10 mg/mL Parsol® MCX, confirment l'hypothèse selon laquelle la quantité de polymère utilisé est un paramètre important. Cependant, une valeur élevée de la tension superficielle de l'ordre de 60 mN/m a été enregistrée pour le système contenant 50 mg/mL de poly-β-cyclodextrine et moins élevée, de l'ordre de 13 mN/m pour le système contenant 100 mg/mL de poly-β-cyclodextrine. Ceci indique la forte stabilité des émulsions contenant plus de polymère (poly-β-cyclodextrine). En effet, l'augmentation de la stabilité des émulsions dans le temps est observée avec l'augmentation de la quantité de polymère dans les systèmes émulsionnants.

Ainsi, pour des essais effectués avec 100 mg/mL de polymère (le système le plus stable dans le temps), on constate par analyse de microscopie optique et diffraction dynamique de la lumière (DLS), la présence des gouttelettes de taille de taille constante et une morphologie stable dans le temps (Figures 4, 5 et 6).

Un autre paramètre important étudié a été le rapport Cyclodextrine /Parsol® MCX avec une variation dans la concentration de Parsol® MCX (Figure 7). On observe dans ce cas une faible augmentation de la taille des gouttelettes jusqu'à 12 µm pour une concentration en Parsol® MCX de 500 mg/mL. Concernant ce système, la présence d'une goûte d'huile surnageante est observée à partir du premier jour pour les concentrations très élevées de Parsol® MCX de 250 et 500 mg/mL. Cependant pour 50 mg /mL de Parsol® MCX (5 %) aucune goutte d'huile n'est visible à la surface d'émulsion ce qui indique une incorporation totale de l'huile (Parsol® MCX). Par ailleurs, l'analyse par diffraction dynamique de la lumière (DLS) sur une solution de poly-beta-cyclodextrine met en évidence la présence d'une population de taille de particule de diamètre très faible de l'ordre de 7 (nm). Ceci permet d'envisager la stabilisation d'une goutte d'huile de grande taille par plusieurs particules de petites tailles de poly-beta-cyclodextrines.

### EXEMPLE 3 : préparation d'émulsions (formulations) cosmétiques (huiles essentielles, parfums, anti-moustique) par voie directe (huile-dans-l'eau) selon le procédé de la présente invention.

Des émulsions contenant des huiles essentielles ou parfums sont préparées selon un protocole similaire à celui de l'Exemple 2. Les huiles et les parfums suivants ont été utilisés dans une quantité égale à 10 mg pour un volume total de 2 mL d'émulsion et pour une concentration en polymères de β-cyclodextrines égale à 100 mg/mL :
- l'huile essentielle de lavandin,
- l'huile de bourrache,
- l'huile de gingembre,
- géraniol,
- concentré de parfum.

Pour chaque composé lipophile, l'émulsion est préparée par sonication pendant 40 secondes ou par ultra-turrax pendant 2 h, jusqu'à l'obtention d'une émulsion homogène. On obtient ainsi une émulsion qui est stable.

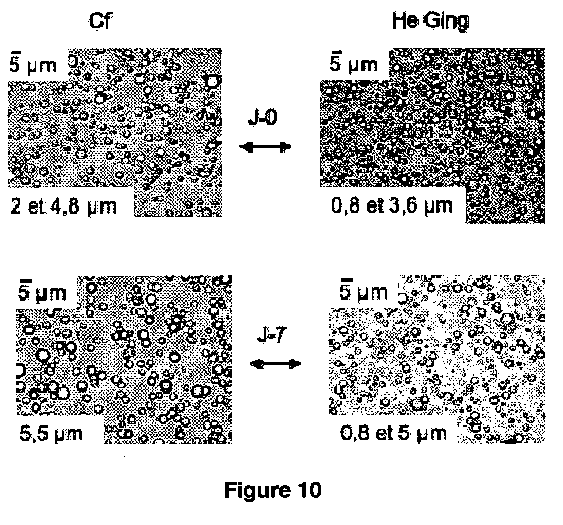

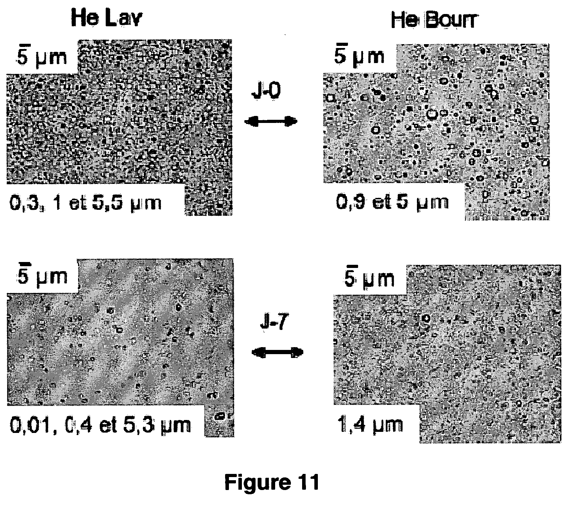

L'examen au microscope optique ainsi par les techniques de diffusion dynamique de la lumière (DLS, granulométrie laser) montrent que les émulsions ainsi préparées forment des gouttelettes sphériques, très bien définies, avec une dimension < 10 µm (Figures 10-12).

Généralement, une grande variété de produits est utilisée pour stabiliser les systèmes émulsionnants : les tensioactifs, les co-tensioactifs, solvants organiques, polymères réticulés. Toutefois tous ces produits présentent un risque de toxicité ou pollution environnementale lié à leurs méthodes de fabrication (synthèse organique lourde). De ce fait, les efforts sont tournés vers la chimie « verte », dans l'utilisation de produits moins polluants, plus pratique, d'origine naturelle et tout en évitant toute formulation d'émulsions à base de tensioactifs, co-tensioactifs, solvants organiques, etc.

Il ressort des exemples précédents que le procédé de fabrication de l'invention permet de formuler de façon très simple une émulsion stable huile-dans -l'eau par un processus « vert » d'encapsulation sans l'utilisation des tensioactifs, des co-tensioactifs et /ou des solvants organiques.

### EXEMPLE 4 : méthodes de concentrations des émulsions selon le procédé de la présente invention

Une crème concentrée de Parsol® MCX a été préparée à partir d'une émulsion de Parsol® MCX de volume total de 2 mL, fraichement préparée, pour une concentration de poly-beta-cyclodextrine de 100 mg/mL et contenant 10 mg/mL Parsol® MCX (rapport molaire 1/ 2,5 beta-cyclodextrine/Parsol® MCX). Après la préparation de l'émulsion conformément à l'EXEMPLE 2, on procède à l'ultracentrifugation à 40.000 rpm pendant 15 minutes. On obtient ainsi après la séparation de la phase inférieure, une crème d'un volume de 0,2 mL ce qui est équivalent à une augmentation de 10 fois de la concentration du Parsol® MCX dans la formulation (100 mg/mL). Ce résultat a été confirmé par dosage en UV-VIS de la phase inférieure ou aucune trace de principe actif (Parsol® MCX) n'a été observée.

L'observation par microscopie optique de la crème de Parsol® MCX obtenue après ultracentrifugation et redispersion nous montre que la taille de gouttelette dans la crème et l'émulsion après redispersion (2 et 4,9 µm) est équivalente à celle de l'émulsion initiale (∼ 2 et 5 µm) (Figures 14-a et b).

Un autre exemple de concentration a été réalisé avec le Géraniol introduit dans le même type de système (100 mg/mL poly-β-cyclodextrine et 11,4 mg/mL Géraniol). Après ultracentrifugation, un volume de 1 mL relatif à la phase concentrée supérieure (crème) a été séparé pour une concentration finale dans le système de 57 mg/mL (récupération de la totalité de quantité de Géraniol introduite).

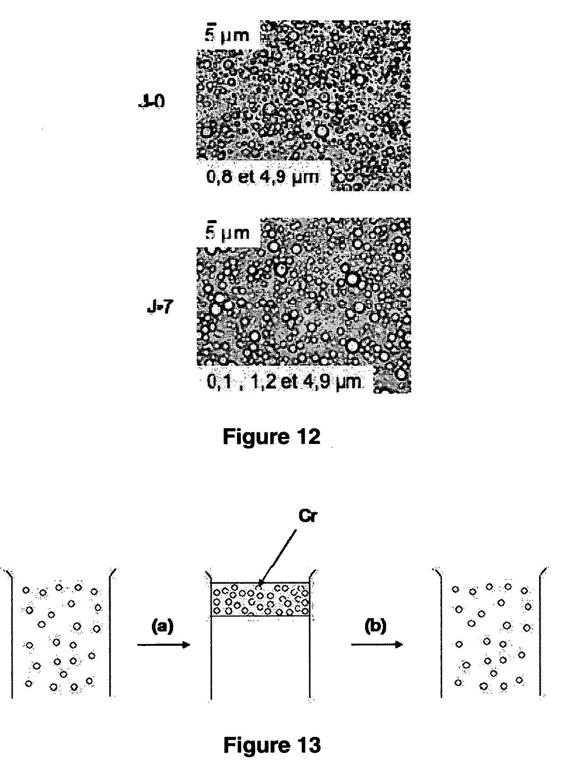

Chaque émulsion préparée dans les Exemples 2 et 3 peut être ultracentrifugée à 40.000 rotations par minutes (rpm) pendant une période de temps allant de 5 minutes à 1 h et de préférence à 15 minutes. Il est ainsi possible de récupérer la crème concentrée et de procéder à une redispersion conformément au schéma décrit dans la Figure 13.

### EXEMPLE 5 : préparation d'émulsions (formulations) antisolaires (Parsol® MCX) par voie indirecte (eau-dans-l'huile) selon le procédé de la présente invention.

Des émulsions par voie indirecte correspondant à une quantité totale égale à 2mL d'émulsion ont été préparées en utilisant des polymères à base de β-cyclodextrines de masse molaire 10⁵ g/mol selon le protocole suivant :
10 mg de poly-β-CD sont solubilisés dans 100 mL d'eau. 2 mL de l'agent anti-UV Parsol® MCX sont ajoutés à 2 mL de la solution aqueuse de poly-β-CD ainsi préparée. L'émulsion est formée par sonication pulsée pendant une période de 40 secondes.

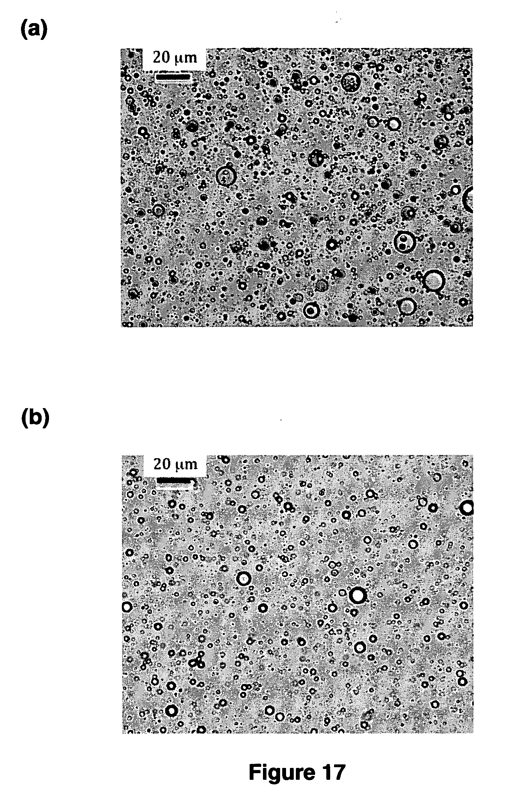

L'examen au microscope optique (observation de la morphologie et analyse d'image) montre que les émulsions ainsi préparées forment des gouttelettes sphériques, très bien définies, avec une dimension < 10 µm (Figure 17).

### EXEMPLE 6: Lyophilisation d'émulsions selon l'invention

Des émulsions par voie directe (huile-dans-l'eau) sont préparées selon les Exemples 2 à 4 ci-dessus. Les récipients les contenant sont ensuite plongés dans l'air liquide. Les émulsions sont ensuite lyophilisées pendant 72 heures (BIOBLOCK SCIENTIFIC CHRIST ALPHA 1-4, vide 15 Pa).

Des émulsions par voie indirecte (eau-dans-l'huile) correspondant à une quantité totale égale à 2 mL d'émulsion ont été préparées en utilisant des polymères à base de β-cyclodextrines de masse molaire 10⁵ g/mol:
200 mg de poly-β-CD sont solubilisés dans 2 mL d'eau (100 mg/mL).
20 mg/mL de l'agent anti-UV (Parsol® MCX) est ajoutée (10 mg/mL).

Ces émulsions ont été lyophilisées.

Les lyophilisats obtenus ont ensuite été redispersés avec 2 mL d'eau milliQ, de façon à reconstituer les émulsions.

Les diamètres des gouttelettes avant et après lyophilisation ont été mesurés : la taille de gouttelette est équivalente à celle de l'émulsion initiale (∼ 2 et 5 µm).

### EXEMPLE 7 : préparation d'émulsions (formulations) à base de Squalène, Huile de Bourrache et huile de Gingembre par voie directe (huile-dans-l'eau), selon le procédé de la présente invention et conservation des échantillons à long terme.

Des émulsions par voie directe correspondant à une quantité totale égale à 2 mL d'émulsion ont été préparées en utilisant des polymères à base de β-cyclodextrines (poly-β-CD) de masse molaire moyenne 10⁵ g/mol selon le protocole suivant :
- 100 mg/mL de (poly-β-CD) et contenant, respectivement:
   - 10 mg/mL Squalène (émulsion (a)),
   - 10 mg/mL Huile de Bourrache (émulsion (b))
   - 10 mg/mL Huile de Gingembre (émulsion (c)),

Les émulsions (a) à (c) sont formées par sonication pulsée pendant une période de 40 secondes et conservées à 25°C.

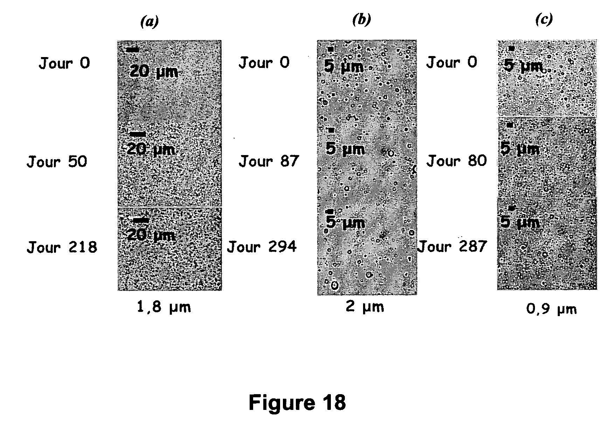

L'observation par microscopie optique des ces émulsions obtenues après sonication, nous montre que la taille de gouttelette est environ de 2 µm. Tous ces types d'émulsions présentent une stabilité importante dans le temps (Figure 18).

Le comportement élastique d'interface (huile/solution aqueuse de polymère) est mesuré à partir de la variation de la tension interfaciale. L'appareil utilisé pour la mesure des propriétés élastiques est un Tracker®, fabriqué par la société Teclis-IT Concept. La tension interfaciale est mesurée par la méthode de la goutte. Une déformation de l'aire interfaciale par dilatation ou par contraction du volume de la goutte engendre une variation de la concentration interfaciale des polymères adsorbés à l'interface. Le Tracker® enregistre la réponse de la tension interfaciale et établit ensuite la relation entre la déformation de l'interface et cette réponse qui décrit le comportement rhéologique de l'interface. Une déformation de l'aire interfaciale est répétée au cours du temps en imposant une variation sinusoïdale du volume de la goutte et permet ainsi d'établir les caractéristiques viscoélastiques de l'interface. L'évolution de ces caractéristiques permet de prévoir l'évolution de la stabilité de l'émulsion et de mettre en évidence un grand nombre de phénomènes physicochimiques. La goutte est formée à l'extrémité du capillaire relié à une seringue dont le piston est commandé par un moteur. L'aiguille utilisée est recourbée à 180° afin d'avoir une goutte montante, la densité des huiles étant ici plus faible que celle de la phase aqueuse. La jauge de l'aiguille est de 18, ce qui correspond à un diamètre intérieur de 0,84 mm et un diamètre extérieur de 1,27 mm. L'aiguille est en téflon, afin d'éviter le mouillage de la goutte sur les parois de l'aiguille. Une goutte d'huile est formée dans la phase aqueuse de polymères permettant d'étudier la tension interfaciale. Le volume initial de cette goutte est fixé. La forme de la goutte résulte de l'équilibre entre forces capillaires et forces de gravité. (Benjamins J.; Cagna A.; H., L.-R. E., Coll. Surf. A. 1996, 114, 245-254 **[27]).**

Des études rhéologiques comparatives (rhéologie interfaciale) sur ce système contenant 24,3 mg/mL et 100 mg/mL de poly-β-CD ainsi que 17 mg/mL de monomère-CD confirment l'hypothèse selon laquelle la quantité de polymère utilisée n'est plus un paramètre important (Voir Exemple comparatif 2 réalisé avec le Parsol® MCX).

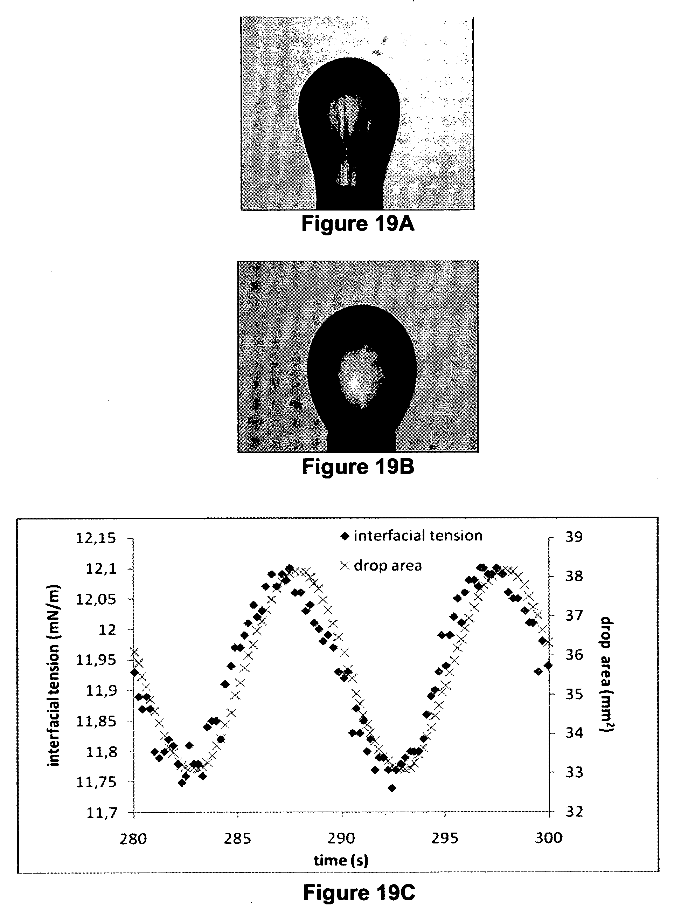

Dans le même contexte, on observe la formation d'une membrane rigide à la surface de la goutte de Squalène dans les cas d'essais effectués avec une solution aqueuse de monomères-β-CD (Fig. 19). Ce fait indique la formation d'un précipité. Cependant, un caractère élastique à l'interface des poly-β-CD et Squalène/Bourrache et Gingembre a été observé. Ceci indique la forte stabilité des émulsions contenant plus de polymère (poly-β-CD) et les trois huiles (Squalène. Bourrache, Gingembre). Par exemple, dans les cas des essais réalisés avec le Squalène, la tension interfaciale est d'approximativement 12 mN/m. On observe dans ce cas l'augmentation de la stabilité des émulsions dans le temps.

### EXEMPLE 8: préparation d'émulsions à base de Sébum synthétique et polymères de cyclodextrins (α et β) par voie directe (huile-dans-eau) selon le procédé de la présente invention.

Des émulsions contenant du sébum synthétique sont préparées selon un protocole similaire à celui de l'Exemple 7.

Une quantité de 750 mg de sébum synthétique à été synthétisée avec la composition suivante, selon le protocole décrit dans GuangWei Lu*, Satyanarayana Valiveti, Julie Spence, Christine Zhuang, Lora Robosky,KimberlyWade, Ann Love, Lain-Yen Hu, David Pole, Matt Mollan, « Comparison of artificial sebum with human and hamster sebum samplesComparison of artificial sebum with human and hamster sebum samples », International Journal of Pharmaceutics 367 (2009) 37-43 **[28] :**
15 % Squalène, 10 % palmitic acid palmitic ester, 10 % oleic acid palmitic ester, 20 % tripalmitin, 20 % triolein, 6 % acide oleic, 10 % acide palmitic, 4 % myristic acide, 3 % cholesterol, 2 % cholesterol oleate (tous les produits sont de provenance de Sigma-Aldrich).

Plusieurs essais d'émulsification du sébum synthétique, correspondant à une quantité totale égale à 2 mL d'émulsion, ont été conduits avec les polymères à base de CD (α et P) de manière suivante :
100 mg/mL de poly-β-CD ou poly-α-CD et contenant des quantités variables de sébum synthétique :
   - 50 mg/mL (5 %),
   - 100 mg/mL (10 %).

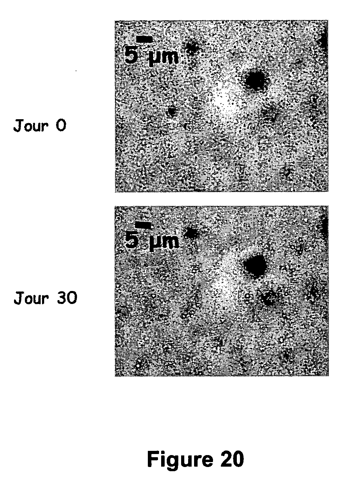

Les émulsions sont formées par sonication pulsée pendant une période de 40 secondes et conservées à 25°C. L'observation par microscopie optique des émulsions obtenues après sonication, nous indique une taille moyenne de gouttelette d'environ 2 µm (Figure 20).

### EXEMPLE 9 : préparation d'émulsions à base de Squalène en mélange avec diverses huiles essentielles (huile de géranium, linalol et lemon-grass) et polymères de cyclodextrines (a et β) par voie directe (huile-dans-l'eau) selon le procédé de la présente invention.

Des émulsions par voie directe selon un protocole similaire à celui de l'Exemple 7, correspondant à une quantité totale égale à 2 mL d'émulsion ont été préparées en utilisant des polymères à base de α-cyclodextrines (poly-α-CD) et de masse molaire 10⁵ g/mol contenant:
- mélange 50/50 (w/w) squalène/ huile de géranium,
- mélange 50/50 (w/w) squalène/ linalol,
- mélange 50/50 (w/w) squalène/ huile essentielle de citronelle

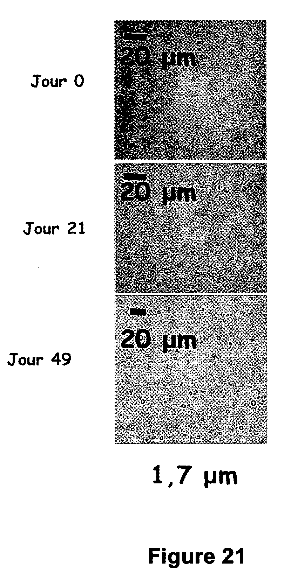

Les émulsions sont formées par sonication pulsée pendant une période de 40 secondes et conservées par agitation tangentielle à 25°C. L'observation par microscopie optique des émulsions obtenues après sonication, nous montre que la taille moyenne de gouttelette est d'environ 2 µm (Figure 21).

### EXEMPLE 10 : test en double aveugle : nettoyage de la peau après rajout de Squalène

Des tests de double aveugle de nettoyage de la peau après rajout d'une goutte de Squalène (20 µL) ont été réalisés avec trois solutions (0,5 mL pendant 20 secondes de nettoyage) :
(a) eau,
(b) solution de monomère β-CD (17 mg/mL, limité par sa solubilité),
(c) solution de poly-β-CD (24,3 mg/mL, le poly-β-CD ayant un % massique en cyclodextrines de 70 % CD ; il s'agit donc d'une teneur en poids de motifs β-CD équivalente à celle du produit (b) dans lequel 17 mg/mL de monomère β-CD sont utilisés).

Les trois solutions ont été testées par un groupe de 10 volontaires âgés de 25 et 45 ans après rajout d'une goutte de Squalène sur la peau du dos de la main. Le nettoyage de la peau a été évalué par les volontaires au choix selon les trois critères suivants :
(1) sensation huileuse,
(2) sensation moins huileuse,
(3) sensation agréable.

Toutes les réponses obtenues montrent le nettoyage rapide et une sensation plus agréable avec la solution de polymère de β-cyclodextrines (Tableau 1).

**Tableau 1 : Classement des échantillons dans le cadre du test double aveugle-nettoyage de la peau après rajout de Squalène:**

| *Numéro échantillon* | *Composition des échantillons* | *Critère du classement* |
|---|---|---|
| (a) | eau | (1) |
| | | (10 réponses sensation huileuse) |
| (b) | solution de monomère β-CD (17 mg/mL) | (2) |
| | | (10 réponses sensation moins huileuse) |
| (c) | solution de poly-β-CD (24,3 mg/mL) | (3) |
| | | (2 réponses sensation moins huileuse) (8 réponses sensation agréable). |

### EXEMPLE 11 : test en double aveugle : masquage d'odeur huile de Bourrache.

Des tests de double aveugle de masquage d'odeur de l'huile de Bourrache, ont été réalisés avec 4 échantillons différents pour 2 mL volume total préparés:
(a) Huile de Bourrache (échantillon pur),
(b) Mélange monomère β-CD (17 mg/mL) solution aqueuse et 10 mg/mL l'huile de Bourrache,
(c) Mélange poly-β-CD (24,3 mg/mL) solution aqueuse et 10 mg/mL l'huile de Bourrache,
(d) Poly-β-CD (solide) et 10 mg/mL l'huile de Bourrache.

Les 4 échantillons ont été testés par un groupe de 8 volontaires âgés entre 25 et 50 ans. Le masquage d'odeur a été noté conformément aux critères suivants :
(1) odeur très persistante,
(2) odeur persistante,
(3) odeur moins persistante,
(4) très peu d'odeur,
(5) pas d'odeur.

Toutes les réponses obtenues montrent le nettoyage rapide et une sensation plus agréable avec la solution de poly-β-CD.

Les 4 échantillons ont été classés de la manière suivante (Tableau 2) :

**Tableau 2 : Classement des échantillons dans le cadre du teste double aveugle - masquage d'odeur huile de Bourrache :**

| *Echantillon* | *Composition des échantillons* | *Critère de classement* |
|---|---|---|
| (a) | Huile de Bourrache (échantillon pur) | (2) |
| | | (5 réponses odeur persistante), (3 réponses odeur moins persistante) |
| (b) | Mélange Monomère β-CD (17 mg/mL) sol. aq. et 10 mg/mL l'huile de Bourrache | (3) |
| | | (7 réponses odeur moins persistante), (1 réponse très peu d'odeur) |
| (c) | Mélange poly-β-CD (24,3 mg/mL) sol. aq. et 10 mg/mL l'huile de Bourrache | (4) |
| | | (7 réponses très peu d'odeur, 1 réponse odeur moins persistante) |
| (d) | poly-β-CD (solide) et 10 mg/mL l'huile de Bourrache. | (4) |
| | | (7 réponses très peu d'odeur, 1 réponse odeur moins persistante) |

### LISTES DES RÉFÉRENCES

[1] EP 0 685 227
[2] FR 2 858 777
[3] Duchêne et al., « Cyclodextrins and emulsions », International Journal of Pharmaceutics, 266 (2003), 85-90
[4] Yu et al., « Effect of camphor/cyclodextrin complexation on the stability of O/W/O multiple emulsions », International Journal of Pharmaceutics, 261 (2003), 1-8
[5] Inoue et al., « Emulsion preparation using β- cyclodextrin and its derivates acting as an emulsifier », Chem. Pharm. Bull, 56 (9), (2008), 1335- 1337
[6] Inoue et al., "Formulation and characterisation of emulsions using β-cyclodextrin as an emulsifier", Chem. Pharm. Bull, 56 (5), (2008), 668- 671
[7] Inoue et al., "Preparation and characterisation of n-alkane / water emulsion stabilized by cyclodextrine", Journal of oleo science, 58, (2), (2009), 85-90
[8] WO 2008/003685
[9] E. Renard et al., European Polymer Journal, vol. 33, No 1, pp 49-57 (1997)
[10] Gref et al., International Journal of Pharmaceutics, Vol. 332, Issues 1-2, Pages 185-191 (2007)
[11] Gref et al., J. Control Release, 111(3):316-24 (2006).
[12] Gref et al., Journal of colloid and interface science, 307(1):83-93 (2007)
[13] Blanchemain et al., Acta Biomaterialia, Volume 4, Issue 6, November 2008, Pages 1725-1733
[14] Elif Yilmaz Ozmen et al. Bioresource Technology, Volume 99, Issue 3, Pages 526-531 (2008)
[15] Cesteros et al., European Polymer Journal, Volume 45, Issue 3, Pages 674-679 (2009)
[16] Salmaso et al., International Journal of Pharmaceutics, Volume 345, Issues 1-2, Pages 42-50 (2007)
[17] Yang et al., Biomaterials, Volume 28, Issue 21, Pages 3245-3254 (2007)
[18] WO 2006/124801
[19] Prabaharan et al., International Journal of Biological Macromolecules, Volume 44, Issue 4, Pages 320-325(2009*)*
[20] Zhang et al., "Chitosan bearing pendant cyclodextrin as a carrier for controlled protein release ", Carbohydrate Polymers, In Press, Corrected Proof, Available online 30 January 2009
[21] Prabaharan et al., Carbohydrate Polymers, Volume 73, Issue 1, Pages 117-125 (2008)
[22] Prabaharan et al., Carbohydrate Polymers, Volume 73, Issue 1, Pages 117-125 (2008)
[23] Lu et al., European Polymer Journal, Volume 44, Issue 7, Pages 2140-2145 (2008)
[24] Blanchemain et al., Acta Biomaterialia, Volume 4, Issue 5, Pages 1392-1400 (2008)
[25] Zha et al., Journal of Membrane Science, Volume 321, Issue 2, Pages 316-323 (2008)
[26] "Cyclodextrins and their inclusion complexes", Szejtli J., Academia Kiado, Budapest, 1982
[27] Benjamins J.; Cagna A.; H., L.-R. E., Coll. Surf. A. 1996, 114, 245-254.
[28] GuangWei Lu*, Satyanarayana Valiveti, Julie Spence, Christine Zhuang, Lora Robosky,KimberlyWade, Ann Love, Lain-Yen Hu, David Pole, Matt Mollan, « Comparison of artificial sebum with human and hamster sebum samplesComparison of artificial sebum with human and hamster sebum samples », International Journal of Pharmaceutics 367 (2009) 37-43.

## Revendications

1. Emulsion simple ou multiple comprenant un complexe d'inclusion non-covalent et non-cristallin formé (i) d'un polymère à base d'unités cyclodextrine ou d'un polymère hydrophile portant des cyclodextrines et (ii) d'un composé lipophile, dans laquelle le polymère à base d'unités cyclodextrine et le polymère hydrophile portant des cyclodextrines contiennent au moins 10 unités cyclodextrine.

2. Emulsion selon la revendication 1, dans laquelle le polymère est choisi dans le groupe comprenant:
- les poly-α, poly-β ou poly-γ-cyclodextrines,
- les copolymères de α, β et/ou γ-cyclodextrines,
- les polymères naturels ou synthétiques sur lesquels sont greffées des α, β et/ou γ-cyclodextrines,
- ou un mélange de ceux-ci ;
dans lesquels les unités α, β et/ou γ-cyclodextrines sont éventuellement modifiées.

3. Emulsion selon l'une quelconque des revendications 1 à 2, dans laquelle le polymère à base d'unités cyclodextrine comporte en moyenne entre 10 et 1000 unités cyclodextrine au sein de sa structure.

4. Emulsion selon l'une quelconque des revendications 1 à 3, dans laquelle les unités cyclodextrine du polymère cyclodextrine sont liées entre elles par des chaînes hydrocarbonées répondant à la formule -O-(CH₂-CHOR¹-CH₂)ₙ-O- où n est un entier compris entre 1 et 50 et, dans chacune des unités (CH₂-CHOR¹-CH₂), R¹ désigne soit un atome d'hydrogène, soit une chaine -CH₂-CHOH-CH₂-O- reliée à une unité cyclodextrine du polymère.

5. Emulsion selon l'une quelconque des revendications 1 à 4, dans laquelle le polymère à base d'unités cyclodextrine est obtenu par polycondensation de molécules de cyclodextrine et d'épichlorhydrine.

6. Emulsion selon l'une quelconque des revendications 1 à 4, dans laquelle le polymère à base d'unités cyclodextrine a une masse molaire moyenne comprise entre 10 000 et 3 000 000 g/mole.

7. Emulsion selon l'une quelconque des revendications 1 à 4, dans laquelle le polymère à base d'unités cyclodextrine est un poly-β-cyclodextrine répondant à la structure suivante: 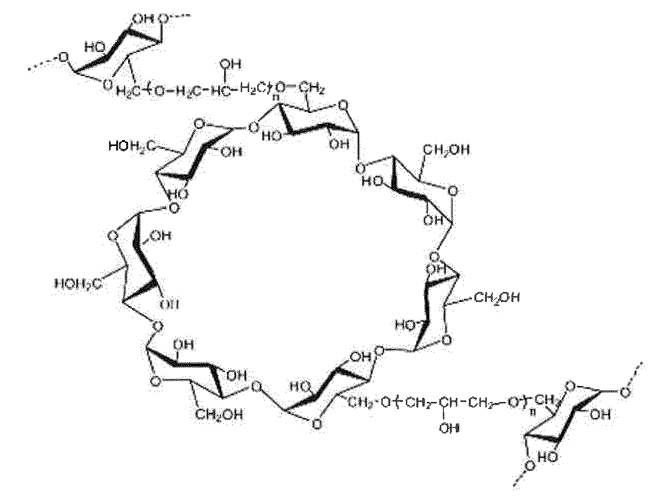 dans laquelle n est un entier compris entre 1 et 50 ; et le nombre d'unités beta-cyclodextrine est en moyenne compris entre 10 et 2000 unités, de préférence en moyenne entre 100 et 1800 unités, de préférence en moyenne entre 500 et 1600 unités, et avantageusement en moyenne entre 800 et 1500 unités.

8. Emulsion selon l'une quelconque des revendications 1 à 4, dans laquelle le polymère hydrophile est un polysaccharide choisi parmi l'acide hyaluronique , l'acide alginique, le chitosane, la chitine, le scleroglucane, le dextrane, l'amilose, l'amilopectine, , un dérivé de la cellulose, l'amidon, le pullulane, la pectine, un alginate, l'héparine, l'ulvane, un caragheenane, le fucane, le curdlan, le xylane, l'acide polyguluronique, le xanthane, l'arabinane, et l'acide polymannuronique.

9. Emulsion selon l'une quelconque des revendications 1 à 8, dans laquelle le composé lipophile est un composé choisi dans le groupe comprenant:
- les corps gras choisis dans le groupe comprenant les huiles naturelles d'origine végétale, animale ou marine, les huiles synthétiques, les huiles minérales, les huiles hydrogénées, l'huile de silicone, les composés hydrocarbonés, les acides gras saturés ou insaturés, les esters d'acide gras, les cires, les alcools gras, les beurres, les cire-esters, ou un mélange de ceux-ci,
- les composés odorants lipophiles utilisés dans la fabrication des parfums, et
- les principes actifs lipophiles.

10. Emulsion selon la revendication 9, dans laquelle le composé lipophile est une huile essentielle d'origine végétale, animale ou marine choisie dans le groupe comprenant l'huile d'olive, l'huile de sésame, l'huile d'argan, l'huile de palme, l'huile de soja, l'huile de pastel, l'huile de tortue, l'huile de babassu, l'aloe vera, l'huile d'avocat, l'allantoïne, le bisabol, l'huile de pépins de raisin, l'huile d'abricot, l'huile de germe de blé, l'huile d'amande, l'huile d'arachide, l'huile de noix de macadamia, l'huile d'argousier, l'huile d'onagre, l'huile de bourrache, l'huile de gingembre, le géraniol, l'huile de jujube, l'huile de vison et la lanoline ; une huile minérale choisie dans le groupe comprenant l'isohexadécane, l'isoparaffin, la ceresin, et la vaseline ; un composé hydrocarboné choisi dans le groupe comprenant les terpènes et le squalène ; l'acide myristique en tant qu'acide gras ; une cire choisie dans le groupe comprenant la cire de baleine, la cire d'abeille et l'huile de jojoba ; un alcool gras choisi dans le groupe comprenant l'alcool myristylique, l'alcool cétylique, l'alcool stéarylique, l'alcool myricylique ; un composé odorant utilisé dans la formulation des parfums ; ou un principe actif lipophile choisi parmi les agents émollients, les agents anti-infectieux, les anticancéreux, les anti-inflammatoires, les anti-bactériens, les antifongiques, les anti-viraux, les anti-séborrhéiques, les anti-acnéiques, les anti-parasitaires, les opioïdes, les kératolytiques, les anti-histaminiques, les anesthésiques, les agents cicatrisants, les modificateurs de la pigmentation, les filtres solaires, les piégeurs de radicaux libres, les agents hydratants, les vitamines, des enzymes, ou encore des polypeptides.

11. Emulsion selon la revendication 10, dans laquelle le composé lipophile est un agent anti-UV organique.

12. Composition comprenant une émulsion telle que définie dans l'une quelconque des revendications 1 à 11.

13. Procédé de préparation d'une émulsion telle que définie dans la revendication 1 **caractérisé en ce qu'**il comprend :
(i) ajouter un composé lipophile dans une solution aqueuse d'un polymère à base d'unités cyclodextrine ou d'un polymère hydrophile portant des cyclodextrines, dans laquelle le polymère à base d'unités cyclodextrine et le polymère hydrophile portant des cyclodextrines contiennent au moins 10 unités cyclodextrine;
(ii) former une émulsion du mélange obtenu à l'étape (i).

14. Procédé selon la revendication 13, dans lequel la formation de l'émulsion de l'étape (ii) est réalisée par un moyen choisi dans le groupe comprenant la sonication, l'homogénéisation par forces de cisaillement, et la microfluidisation.

15. Procédé selon l'une quelconque des revendications 13 à 14, dans lequel le composé lipophile est ajouté en tant que tel lorsqu'il se présente sous forme liquide, ou, lorsqu'il se présente sous forme solide, est ajouté en solution dans un solvant lipide non miscible à l'eau.

16. Procédé selon la revendication 15, dans lequel le solvant lipide est le myristate d'isopropyle ou le squalène ; ou bien un corps gras liquide choisis dans le groupe comprenant les huiles naturelles d'origine végétale, animale ou marine, les huiles synthétiques, les huiles minérales, les huiles hydrogénées, l'huile de silicone, les composés hydrocarbonés, les acides gras saturés ou insaturés, les esters d'acide gras, les cires liquides, les alcools gras, ou un mélange de ceux-ci.

17. Procédé selon l'une quelconque des revendications 13 à 16, dans lequel le polymère est tel que défini dans l'une quelconque des revendications 2 à 8.

18. Procédé selon l'une quelconque des revendications 13 à 16, dans lequel la concentration du polymère à unités cyclodextrine dans la solution aqueuse de l'étape (i) est comprise entre 20 et 200 mg/ml.

19. Procédé selon l'une quelconque des revendications 13 à 18, dans lequel le composé lipophile est tel que défini dans l'une quelconque des revendications 9 à 11.

20. Procédé selon l'une quelconque des revendications 13 à 19, dans lequel, dans l'étape (i), la quantité de composé lipophile est comprise entre 1 et 500 mg/ml.

21. Procédé selon l'une quelconque des revendications 13 à 20, comprenant en outre une étape d'augmentation de la teneur en composé lipophile de l'émulsion par ultracentrifugation de ladite émulsion.

22. Procédé selon l'une quelconque des revendications 13 à 21, comprenant en outre une étape de lyophilisation de l'émulsion.

23. Emulsion obtenue par un procédé selon l'une quelconque des revendications 13 à 22.

24. Utilisation d'une émulsion obtenue par un procédé selon la revendication 21 pour réaliser une encapsulation concentrée de composés lipophiles.

25. Utilisation d'une émulsion selon la revendication 23 pour la fabrication d'une composition cosmétique, pharmaceutique ou agro-alimentaire.

26. Utilisation selon la revendication 25 dans laquelle l'émulsion comprend un agent anti-UV organique comme composé lipophile, pour la fabrication de compositions pour la protection de la peau et/ou les cheveux contre le rayonnement ultraviolet, en particulier le rayonnement solaire.

27. Utilisation d'un complexe d'inclusion non-covalent et non-cristallin formé (i) d'un polymère à base d'unités cyclodextrine ou d'un polymère hydrophile portant des cyclodextrines et (ii) d'un composé lipophile, pour stabiliser une émulsion, dans lequel complexe d'inclusion le polymère à base d'unités cyclodextrine et le polymère hydrophile portant des cyclodextrines contiennent au moins 10 unités cyclodextrine.

28. Composition comprenant une émulsion selon la revendication 11 pour son utilisation pour protéger la peau et/ou les cheveux contre le rayonnement ultraviolet, en particulier le rayonnement solaire.

29. Composition obtenue par lyophilisation d'une émulsion telle que définie dans la revendication 23.

30. Emulsion telle que définie dans la revendication 23 pour son utilisation dans la vectorisation de composés lipophiles présentant un effet thérapeutique ou cosmétique, ou à usage agroalimentaire.

31. Utilisation d'une émulsion telle que définie dans la revendication 23 pour :
- atténuer ou masquer l'odeur d'un composé lipophile composant ladite émulsion,
- atténuer ou masquer le mauvais goût d'un composé lipophile composant ladite émulsion,
- améliorer la stabilité des composés lipophiles facilement oxydables,
- améliorer la stabilité des composés lipophiles facilement volatiles, et/ou
- augmenter la solubilité des composés lipophiles insolubles.

## Patentansprüche

1. Einfache oder mehrfache Emulsion, umfassend einen nicht kovalenten und nicht kristallinen Inklusionskomplex, gebildet aus (i) einem Polymer auf der Grundlage von Cyclodextrineinheiten oder einem hydrophilen Polymer mit Cyclodextrinen und (ii) einer lipophilen Verbindung, wobei das Polymer auf der Grundlage von Cyclodextrineinheiten und das hydrophile Polymer mit Cyclodextrinen mindestens 10 Cyclodextrineinheiten enthalten.

2. Emulsion nach Anspruch 1, wobei das Polymer ausgewählt ist aus der Gruppe, bestehend aus:
- den Poly-α-, Poly-β- oder Poly-γ-cyclodextrinen,
- den Copolymeren von α-, β- und/oder γ-Cyclodextrinen,
- den natürlichen oder synthetischen Polymeren, auf die α-, β- und/oder γ-Cyclodextrine implantiert sind,
- oder einer Mischung dieser;
wobei die α-, β- und/oder γ-Cyclodextrineinheiten eventuell modifiziert sind.

3. Emulsion nach einem der Ansprüche 1 bis 2, wobei das Polymer auf der Grundlage von Cyclodextrineinheiten durchschnittlich zwischen 10 und 1000 Cyclodextrine in seiner Struktur umfasst.

4. Emulsion nach einem der Ansprüche 1 bis 3, wobei die Cyclodextrineinheiten des Cyclodextrinpolymers untereinander durch Kohlenwasserstoffketten verbunden sind, die der Formel -O-(CH₂-CHOR¹-CH₂)ₙ-O- entsprechen, wobei n eine ganze Zahl zwischen 1 und 50 ist und, in jeder der Einheiten (CH₂-CHOR¹-CH₂), R¹ entweder ein Wasserstoffatom oder eine Kette-CH₂-CHOH-CH₂-O- bezeichnet, die mit einer Cyclodextrineinheit des Polymers verbunden ist.

5. Emulsion nach einem der Ansprüche 1 bis 4, wobei das Polymer auf der Grundlage von Cyclodextrineinheiten durch Polykondensierung von Cyclodextrin- und Epichlorhydrinmolekülen erhalten wird.

6. Emulsion nach einem der Ansprüche 1 bis 4, wobei das Polymer auf der Grundlage von Cyclodextrineinheiten eine mittlere Molmasse umfasst, die zwischen 10 000 und 3 000 000 g/mol liegt.

7. Emulsion nach einem der Ansprüche 1 bis 4, wobei das Polymer auf der Grundlage von Cyclodextrineinheiten ein Poly-β-cyclodextrin ist, das der folgenden Struktur entspricht: 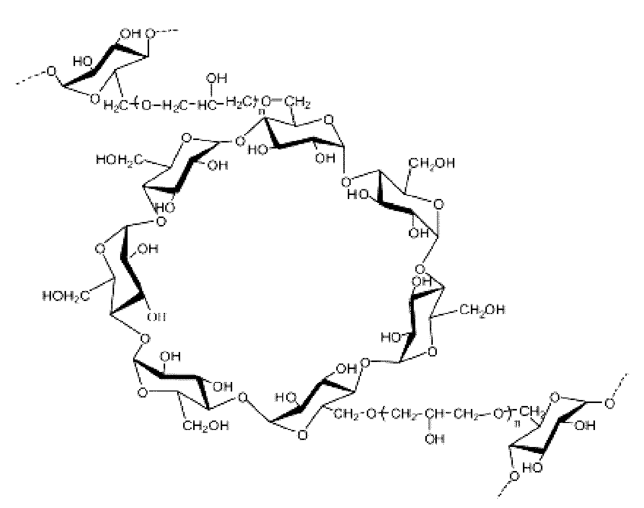 wobei n eine ganze Zahl zwischen 1 und 50 ist; und die Anzahl von beta-Cyclodextrineinheiten durchschnittlich zwischen 10 und 2000 Einheiten liegt, vorzugsweise durchschnittlich zwischen 100 und 1800 Einheiten, vorzugsweise durchschnittlich zwischen 500 und 1600 Einheiten, und vorteilhafterweise durchschnittlich zwischen 800 und 1500 Einheiten.

8. Emulsion nach einem der Ansprüche 1 bis 4, wobei das hydrophile Polymer ein Polysaccharid ist, ausgewählt aus Hyaluronsäure, Alginsäure, Chitosan, Chitin, Scleroglucan, Dextran, Amylose, Amylopektin, einem Derivat der Zellulose, Stärke, Pullulan, Pektin, Alginat, Heparin, Ulvan, Carrageen, Fucan, Curdlan, Xylan, Polyguluronsäure, Xanthan, Arabinan und Polymannuronsäure.

9. Emulsion nach einem der Ansprüche 1 bis 8, wobei die lipophile Verbindung eine Verbindung ist, ausgewählt aus der Gruppe, bestehend aus:
- Fettkörpern, ausgewählt aus der Gruppe, bestehend aus natürlichen Ölen pflanzlichen, tierischen oder marinen Ursprungs, synthetischen Ölen, mineralischen Ölen, hydrierten Ölen, Silikonöl, Kohlenwasserstoffverbindungen, gesättigten und nicht gesättigten Fettsäuren, Fettsäureestern, Wachsen, Fettalkoholen, Buttern, Wachsestern oder einer Mischung dieser;
- lipophile Geruchsverbindungen, die bei der Herstellung von Parfums verwendet werden, und
- lipophile Wirkstoffe.

10. Emulsion nach Anspruch 9, wobei die lipophile Verbindung ein essentielles Öl pflanzlichen, tierischen oder marinen Ursprungs ist, ausgewählt aus der Gruppe, bestehend aus Olivenöl, Sesamöl, Arganöl, Palmöl, Sojaöl, Waidöl, Schildkrötenöl, Babassuöl, Aloe Vera, Avocadolöl, Alantoin, Bisabol, Traubenkernöl, Aprikosenöl, Weizenkeimöl, Mandelöl, Erdnussöl, Macadamianussöl, Sanddornöl, Nachtkerzenöl, Borretschöl, Ingweröl, Geraniol, Jojobaöl, Nerzöl und Lanolin; ein Mineralöl, ausgewählt aus der Gruppe, bestehend aus Isohexadekan, Isoparaffin, Ceresin und Vaselin; eine Kohlenstoffverbindung, ausgewählt aus der Gruppe, bestehend aus Terpenen und Squalen; Myristinsäure als Fettsäure; ein Wachs, ausgewählt aus der Gruppe, bestehend aus Walwachs, Bienenwachs und Jojobaöl; eine Fettalkohol, ausgewählt aus der Gruppe, bestehend aus Myristylalkohol, Cetylalkohol, Stearylalkohol, Myricylalkohol; einerGeruchsverbindung, die bei der Herstellung von Parfums verwendet wird; oder ein lipophiler Wirkstoff, ausgewählt aus Erweichungsmitteln, infektionshemmenden Mitteln, Antikrebsmitteln, Entzündungshemmern, antibakteriellen Mitteln, antimykotischen Mitteln, antiviralen Mitteln, antiseborrhoischen Mitteln, Antiaknemitteln, Schädlingsbekämpfungsmitteln, Opoiden, kieratolytischen Mitteln, Antihistaminika, Anästhetika, Wundheilungsmitteln, Mitteln zur Veränderung der Pigmentierung, Sonnenfiltern, Fängern von freien Radikalen, Feuchthaltemitteln, Vitaminen, Enzymen oder auch Polypeptiden.

11. Emulsion nach Anspruch 10, wobei die lipophile Verbindung ein organisches Anti-UV-Mittel ist.

12. Zusammensetzung, umfassend eine Emulsion, wie in einem der Ansprüche 1 bis 11 definiert.

13. Verfahren zur Herstellung einer Emulsion, wie in Anspruch 1 definiert, **dadurch gekennzeichnet, dass** es Folgendes umfasst:
(i) Zugeben einer lipophilen Verbindung in eine wässrige Lösung eines Polymers auf der Grundlage von Cyclodextrineinheiten oder eines hydrophilen Polymers mit Cyclodextrinen, wobei das Polymer auf der Grundlage von Cyclodextrineinheiten und das hydrophile Polymer mit Cyclodextrinen mindestens 10 Cyclodextrineinheiten enthalten;
(ii) Bilden einer Emulsion der Mischung, erhalten in Schritt (i).

14. Verfahren nach Anspruch 13, wobei die Bildung der Emulsion von Schritt (ii) durch ein Mittel erfolgt, augewählt aus den Gruppe, bestehend aus der Beschallung, der Homogenisierung durch Scherkräfte, und der Mikrofluidisierung.

15. Verfahren nach einem der Ansprüche 13 bis 14, wobei die lipophile Verbindung als solche zugegeben wird, wenn sie in flüssiger Form vorliegt, oder, wenn sie in fester Form vorliegt, als Lösung in ein Lipidlösemittel zugegeben wird, das nicht mit Wasser gemischt werden kann.

16. Verfahren nach Anspruch 15, wobei das Lipidlösemittel Isopropylmyristat oder Squalen ist oder auch ein flüssiger Fettkörper, augewählt aus den Gruppe, bestehend aus natürlichen Ölen pflanzlichen, tierischen oder marinen Ursprungs, synthetischen Ölen, mineralischen Ölen, hydrierten Ölen, Silikonöl, Kohlenwasserstoffverbindungen, gesättigen und nicht gesättigten Fettsäuren, Fettsäureestern, flüssigen Wachsen, Fettalkoholen oder einer Mischung dieser.

17. Verfahren nach einem der Ansprüche 13 bis 16, wobei das Polymer so wie in einem der Ansprüche 2 bis 8 definiert ist.

18. Verfahren nach einem der Ansprüche 13 bis 16, wobei die Konzentration des Polymers mit Cyclodextrineinheiten in der wässrigen Lösung von Schritt (i) zwischen 20 und 200 mg/ml liegt.

19. Verfahren nach einem der Ansprüche 13 bis 18, wobei die lipophile Verbindung so wie in einem der Ansprüche 9 bis 11 definiert ist.

20. Verfahren nach einem der Ansprüche 13 bis 19, wobei in Schritt (i) die Menge der lipophilen Verbindung zwischen 1 und 500 mg/ml liegt.

21. Verfahren nach einem der Ansprüche 13 bis 20, ausserdem umfassend einen Schritt des Erhöhens des Gehalts an lipophiler Verbindung der Emulsion durch Ultrazentrifugieren der Emulsion.

22. Verfahren nach einem der Ansprüche 13 bis 21, ausserdem umfassend einen Schritt des Lyophilisierens der Emulsion.

23. Emulsion, erhalten durch ein Verfahren nach einem der Ansprüche 13 bis 22.

24. Verwendung einer Emulsion, erhalten durch ein Verfahren nach Anspruch 21, um eine konzentrierte Einkapselung der lipophilen Verbindungen durchzuführen.

25. Verwendung einer Emulsion nach Anspruch 23 zur Herstellung einer kosmetischen, pharmazeutischen oder Lebensmittel-Zusammensetzung.

26. Verwendung nach Anspruch 25, wobei die Emulsion ein organisches Anti-UV-Mittel als lipophile Verbindung zur Herstellung von Zusammensetzungen für den Schutz der Haut und/oder der Haare gegen die ultraviolette Strahlung, insbesondere die Sonnenstrahlung, umfasst.

27. Verwendung eines nicht kovalenten und nicht kristallinen Inklusionskomplexes, gebildet aus (i) einem Polymer auf der Grundlage von Cyclodextrineinheiten oder einem hydrophilen Polymer mit Cyclodextrinen und (ii) einer lipophilen Verbindung, um eine Emulsion zu stabilisieren, wobei der Inklusionskomplex das Polymer auf der Grundlage von Cyclodextrineinheiten und das hydrophile Polymer mit Cyclodextrinen mindestens 10 Cyclodextrineinheiten enthalten.

28. Zusammensetzung, umfassend eine Emulsion nach Anspruch 11 für ihre Verwendung, um die Haut und/oder die Haare gegen die ultraviolette Strahlung, insbesondere die Sonnenstrahlung, zu schützen.

29. Zusammensetzung, erhalten durch die Lyophilisierung einer Emulsion, wie in Anspruch 23 definiert.

30. Emulsion wie in Anspruch 23 definiert, für ihre Verwendung bei der Vektorisierung von lipophilen Verbindungen, die eine therapeutische oder kosmetische Wirkung aufweisen, oder zur Verwendung in Lebensmitteln.

31. Verwendung einer Emulsion, wie in Anspruch 23 definiert, um
- den Geruch einer lipophilen Verbindung, die die Emulsion bildet, zu dämpfen oder zu verdecken,
- den schlechten Geschmack einer lipophilen Verbindung, die die Emulsion bildet, zu dämpfen oder zu verdecken,
- die Stabilität der lipophilen Verbindungen, die leicht oxidierbar sind, zu verbessern,
- die Stabilität der lipophilen Verbindungen, die leicht flüchtig sind, zu verbessern, und/oder
- die Löslichkeit der unlöslichen lipophilen Verbindungen zu erhöhen.

## Claims

1. Simple or multiple emulsion comprising a non-covalent and non-crystalline inclusion complex comprising (i) a cyclodextrin unit-based polymer or a hydrophilic polymer bearing cyclodextrins and (ii) a lipophilic compound, wherein the cyclodextrin unit-based polymer and the hydrophilic polymer bearing cyclodextrins contain at least 10 cyclodextrin units.

2. An emulsion according to claim 1, wherein the polymer is selected from the group comprising:
- poly-α-, poly-β- or poly-γ-cyclodextrins,
- copolymers of α-, β- and/or γ-cyclodextrins,
- natural or synthetic polymers onto which α-, β-and/or γ-cyclodextrins are grafted,
- or a mixture thereof;
wherein the α-, β- and/or γ-cyclodextrin units are optionally modified.

3. An emulsion according to any one of claims 1 and 2, wherein the cyclodextrin unit-based polymer comprises, on average, between 10 and 1000 cyclodextrin units within its structure.

4. An emulsion according to any one of claims 1 to 3, wherein the cyclodextrin units of the cyclodextrin polymer are bound to one another via hydrocarbon chains having the formula -O-(CH₂-CHOR¹-CH₂)ₙ-O-, wherein n is an integer between 1 and 50 and, in each of the units (CH₂-CHOR¹-CH₂), R¹ represents either a hydrogen atom or a -CH₂-CHOH-CH₂-O-chain bound to a cyclodextrin unit of the polymer.

5. An emulsion according to any one of claims 1 to 4, wherein the cyclodextrin unit-based polymer is obtained by polycondensation of cyclodextrin and epichlorohydrin molecules.

6. An emulsion according to any one of claims 1 to 4, wherein the cyclodextrin unit-based polymer has an average molar mass between 10 000 and 3 000 000 g/mol.

7. An emulsion according to any one of claims 1 to 4, wherein the cyclodextrin unit-based polymer is a poly-β-cyclodextrin having the following structure: 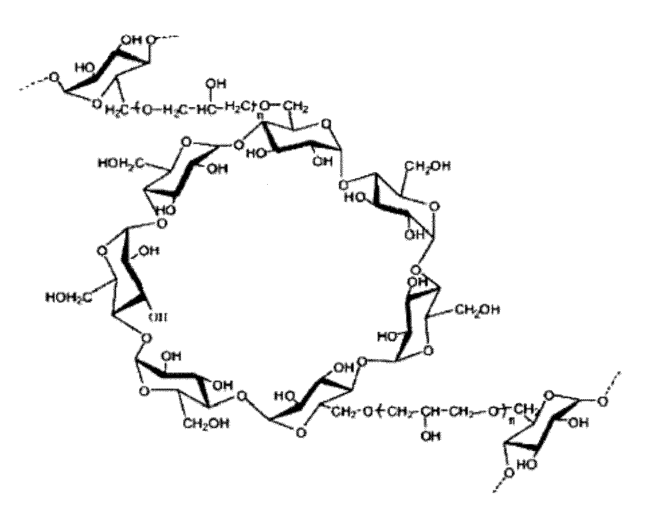 wherein n is an integer between 1 and 50; and the number of beta-cyclodextrin units is, on average, between 10 and 2000 units, preferably, on average, between 100 and 1800 units, preferably, on average, between 500 and 1600 units, and advantageously, on average, between 800 and 1500 units.

8. An emulsion according to any one of claims 1 to 4, wherein the hydrophilic polymer is a polysaccharide selected from hyaluronic acid, alginic acid, chitosan, chitin, scleroglucan, dextran, amylose, amylopectin, a cellulose derivative, starch, pullulan, pectin, an alginate, heparin, ulvan, a carrageenan, fucan, curdlan, xylan, polyguluronic acid, xanthan, arabinan and polymannuronic acid.

9. An emulsion according to any one of claims 1 to 8, wherein the lipophilic compound is a compound selected from the group comprising:
- fatty substances selected from the group comprising natural oils of vegetable, animal or marine origin, synthetic oils, mineral oils, hydrogenated oils, silicone oils, hydrocarbon-based compounds, saturated or unsaturated fatty acids, fatty acid esters, waxes, fatty alcohols, butters, wax esters, or a mixture thereof,
- lipophilic odorous compounds used in the production of fragrances, and
- lipophilic active ingredients.

10. An emulsion according to claim 9, wherein the lipophilic compound is an essential oil of vegetable, animal or marine origin, seelcted from the group comprising olive oil, sesame oil, argan oil, palm oil, soybean oil, woad oil, turtle oil, babassu oil, aloe vera, avocado oil, allantoin, bisabol, grapeseed oil, apricot oil, wheatgerm oil, almond oil, arachis oil, macadamia nut oil, sea buckthorn oil, evening primrose oil, borage oil, ginger oil, geraniol, jujube oil, mink oil and lanolin; a mineral oil selected from the group comprising isohexadecane, isoparaffin, ceresin and petrolatum; a hydrocarbon-based compound selected from the group comprising terpenes and squalene; myristic acid as a fatty acid; a wax selected from the group comprising whale wax, beeswax and jojoba oil; a fatty alcohol selected from the group comprising myristyl alcohol, cetyl alcohol, stearyl alcohol, myricyl alcohol; an odorous compound used in the formulation of fragrances; or a lipophilic active ingredient selected from emollients, anti-infectives, anticancer agents, anti-inflammatories, antibacterial agents, antifungal agents, antivirals, antiseborrheic agents, antiacne agents, antiparasitics, opioids, keratolytics, antihistamines, anesthetics, wound healing agents, pigmentation modifiers, UV-filters, free radical scavengers, moisturizers, vitamins, enzymes, or else polypeptides.

11. An emulsion according to claim 10, wherein the lipophilic compound is an organic anti-UV agent.

12. A composition comprising an emulsion as defined in any one of claims 1 to 11.

13. A method for preparing an emulsion as defined in claim 1, comprising:
(i) adding a lipophilic compound to an aqueous solution of a cyclodextrin unit-based polymer or of a hydrophilic polymer bearing cyclodextrins, wherein the cyclodextrin unit-based polymer and the hydrophilic polymer bearing cyclodextrins contain at least 10 cyclodextrin units;
(ii) forming an emulsion of the mixture obtained in step (i).

14. A method according to claim 13, wherein the formation of the emulsion in step (ii) is carried out by a means selected from the group comprising sonication, homogenization by shear forces, and microfluidization.

15. A method according to any one of claims 13 and 14, wherein the lipophilic compound is added as such when it is in liquid form, or, when it is in solid form, is added in solution in a water-immiscible lipid solvent.

16. A method according to claim 15, wherein the lipid solvent is isopropyl myristate or squalene; or else a liquid fatty substance selected from the group comprising natural oils of vegetable, animal or marine origin, synthetic oils, mineral oils, hydrogenated oils, silicone oil, hydrocarbon-based compounds, saturated or unsaturated fatty acids, fatty acid esters, liquid waxes, fatty alcohols, or a mixture thereof.

17. A method according to any one of claims 13 to 16, wherein the polymer is as defined in any one of claims 2 to 8.

18. A method according to any one of claims 13 to 16, wherein the concentration of the cyclodextrin unit-based polymer in the aqueous solution of step (i) is between 20 and 200 mg/ml.

19. A method according to any one of claims 13 to 18, wherein the lipophilic compound is as defined in any one of claims 9 to 11.

20. A method according to any one of claims 13 to 19, wherein, in step (i), the amount of lipophilic compound is between 1 and 500 mg/ml.

21. A method according to any one of claims 13 to 20, further comprising a step of increasing the lipophilic compound content of the emulsion by ultracentrifuging said emulsion.

22. A method according to any one of claims 13 to 21, further comprising a step of freeze-drying the emulsion.

23. An emulsion obtained by a method of any one of claims 13 to 22.

24. Use of an emulsion obtained by a method of claim 21, for carrying out a concentrated encapsulation of lipophilic compounds.

25. Use of an emulsion according to claim 23, for the manufacture of a cosmetic, pharmaceutical or agri-food composition.

26. Use of an emulsion according to claim 25, wherein the emulsion comprises an organic anti-UV agent as lipophilic compound, for the manufacture of compositions for protecting the skin and/or the hair against ultraviolet radiation, in particular solar radiation.

27. Use of a non-covalent and non-crystalline inclusion complex comprising (i) a cyclodextrin unit-based polymer or a hydrophilic polymer bearing cyclodextrins and (ii) a lipophilic compound, for stabilizing an emulsion, in which inclusion complex the cyclodextrin unit-based polymer and the hydrophilic polymer bearing cyclodextrins contain at least 10 cyclodextrin units.

28. A composition comprising an emulsion according to claim 11 for use in protecting the skin and/or the hair against ultraviolet radiation, in particular solar radiation.

29. A composition obtained by freeze-drying an emulsion as defined in claim 23.

30. Use of an emulsion as defined in claim 23, for use in vectorizing lipophilic compounds having a therapeutic or cosmetic effect, or for agri-food use.

31. Use of an emulsion as defined in claim 23, for:
- toning down or masking the odor of a lipophilic compound contained in said emulsion,
- toning down or masking the unpleasant taste of a lipophilic compound contained in said emulsion,
- improving the stability of readily oxidizable lipophilic compounds,
- improving the stability of readily volatile lipophilic compounds, and/or
- increasing the solubility of insoluble lipophilic compounds.
